# FASCICULE DE BREVET EUROPEEN

(11) **EP 2 648 690 B1**
(45) Date de publication et mention de la délivrance du brevet: **21.03.2018**
(21) Numéro de dépôt: 11799870.8
(22) Date de dépôt: 05.12.2011
(51) Int. Cl.: A61K 8/49, A61Q 19/00

(54) **COMPLEXE SASPASE-FLG2, AGENTS MODULATEURS ET UTILISATIONS**
SASPASE-FLG2-KOMPLEX, MODULIERENDE MITTEL UND VERWENDUNG
SASPASE-FLG2 COMPLEX, MODULATING AGENTS, AND USES

(30) Priorité: 07.12.2010 FR 1060183; 23.12.2010 US 201061457092 P
(43) Date de publication de la demande: 16.10.2013
(73) Titulaire: L'Oréal, 75008 Paris (FR)
(72) Inventeur: BERNARD, Dominique, F-92170 Vanves (FR); THOMAS-COLLIGNON, Agnès, F-78180 Montigny Le Bretonneux (FR); ROZOT, Roger, F-77400 Lagny Sur Marne (FR); MULLER, Benoit, F-75019 Paris (FR)
(74) Mandataire: Nony
(86) Numéro de dépôt international: PCT/IB2011/055455
(87) Numéro de publication internationale: WO 2012/077034

(56) Documents cités:
- WO-A2-2008/008704
- FR-A1- 2 842 209
- AGARWAL A ET AL: "Solid supported synthesis of structurally diverse dihydropyrido[2,3-d]pyrimidines using microwave irradiation", TETRAHEDRON LETTERS, ELSEVIER, AMSTERDAM, NL, vol. 46, no. 8, 21 février 2005 (2005-02-21), pages 1345-1348, XP004756250, ISSN: 0040-4039, DOI: DOI:10.1016/J.TETLET.2004.12.109 cité dans la demande
- MATSUI T ET AL: "Mouse homologue of skin-specific retroviral-like aspartic protease involved in wrinkle formation", JOURNAL OF BIOLOGICAL CHEMISTRY, THE AMERICAN SOCIETY OF BIOLOGICAL CHEMISTS, INC., BALTIMORE, MD, US, vol. 281, no. 37, 15 septembre 2006 (2006-09-15), pages 27512-27525, XP002561740, ISSN: 0021-9258, DOI: DOI:10.1074/JBC.M603559200 [extrait le 2006-07-12]
- W.C.COFFEEN AND T.J.WOLPERT: "Purification and characterization of serine proteases that exhibit caspase-like activity and are associated with programmed cell death in Avena Sativa", THE PLANT CELL, 2004, pages 857-873, XP002649085, Extrait de l'Internet: URL:http://www.plantcell.org/content/16/4/ 857.full.pdf+html [extrait le 2011-07-11]

## Description

La présente invention concerne une nouvelle cible cosmétique ou thérapeutique utile à l'égard de la peau et de ses annexes, ainsi que sa mise en oeuvre à des fins de soin cosmétique, thérapeutique ou dermatologique.

Plus précisément, la présente invention concerne un nouveau complexe protéique impliqué dans la différentiation et/ou la prolifération des cellules de l'épiderme, son utilisation à titre d'outils de criblage de nouveaux actifs cosmétiques ou thérapeutiques, les nouveaux actifs identifiés, et l'utilisation de ces derniers pour prévenir et/ou traiter des défauts esthétiques ou des troubles pathologiques de la peau et/ou de ses annexes.

Par « la peau», on entend l'ensemble de la peau du corps, incluant le cuir chevelu et les muqueuses.

Par « annexes de la peau », on entend les poils, les cils, les cheveux et les ongles.

La peau, ou épiderme, est conventionnellement divisé en une couche basale de kératinocytes constituant la couche germinative de l'épiderme, une couche dite épineuse pluristratifiée constituée de cellules polyédriques disposées sur la couche germinative, une à trois couches dites granuleuses constituées de cellules aplaties contenant des inclusions cytoplasmiques distinctes, les grains de kératohyaline, et enfin, un ensemble de couches supérieures appelées couches cornées, ou *stratum corneum*, constituées de kératinocytes au stade terminal de leur différentiation, appelés cornéocytes.

Les cornéocytes sont des cellules anucléées principalement constituées d'une matière fibreuse contenant des cytokératines, entourées d'une enveloppe cornée. Il y a en permanence production de nouveaux kératinocytes pour compenser la perte en continu de cellules épidermiques au niveau de la couche cornée, selon un mécanisme dénommé desquamation. L'ensemble du processus conduisant à la formation du *stratum corneum* et à son élimination fait l'objet d'un équilibre finement régulé sous le contrôle de multiples facteurs hormonaux ou cellulaires. Un déséquilibre de l'un de ces facteurs, s'il n'est pas rapidement corrigé, finit par se traduire par un déséquilibre entre la production des cellules au niveau de la couche basale, et le taux de desquamation.

De nombreux défauts esthétiques ou troubles pathologiques de la peau ou de ses annexes peuvent être liés à un défaut de l'homéostasie de l'épiderme et en particulier à un défaut de prolifération et/ou de différenciation des cellules.

Ainsi, lorsque la prolifération et/ou la différenciation des cellules de la couche basale de l'épiderme sont accélérées par rapport au taux de desquamation ou que ce dernier ralenti, alors le *stratum corneum* tend à s'épaissir. Dans le cas de manifestations légères de ce déséquilibre, celui-ci peut apparaître sous la forme de différents défauts esthétiques de la peau ou de ses annexes, comme par exemple, des signes cutanés du vieillissement, des défauts de la fonction barrière de la peau, des signes de sécheresse cutanée, ou des signes cutanés d'une hyperséborrhée. En revanche, les manifestations aggravées de ce déséquilibre peuvent apparaître sous la forme de différents troubles pathologiques, comme par exemple, une hyperkératose, une xérose, une ichtyose, le psoriasis, certaines lésions tumorales bénignes ou malignes, ou des hyperkératoses réactionnelles.

A l'inverse, un ralentissement de la prolifération et/ou de la différenciation des cellules de la couche basale par rapport à la desquamation, ou une accélération de la desquamation, peut se manifester par un amincissement de l'épiderme, et plus particulièrement de la couche cornée. Les manifestations légères de ce déséquilibre peuvent s'exprimer par une fragilité excessive du revêtement cutané, voire par différents défauts esthétiques de la peau ou de ses annexes, tel qu'un défaut de cicatrisation, ou un défaut de ré-épithélialisation, notamment après un traitement de gommage cutané ou exfoliant. Les manifestations aggravées de ce déséquilibre peuvent apparaître sous la forme de différents troubles pathologiques, comme par exemple, des réactions d'origine immunitaire, généralement induites par un contact de la peau avec un ou plusieurs agents exogènes.

De nombreux aspects des mécanismes sous-jacent à la régulation de l'équilibre entre prolifération et différentiation des cellules de l'épiderme demeurent à déterminer.

Ainsi, l'identification et la détermination au niveau cellulaire et au niveau moléculaire des mécanismes impliqués dans la formation du *stratum corneum* permettraient de fournir de nouvelles cibles utiles à l'égard du soin cosmétique ou thérapeutique de la peau et/ou de ses annexes.

Il demeure donc un besoin de disposer de nouvelles cibles moléculaires impliquées dans la formation du *stratum corneum*, et notamment dans la prolifération et/ou la différenciation des cellules de l'épiderme.

Il existe encore un besoin de disposer de nouvelles cibles cosmétiques et/ou thérapeutiques impliquées dans les voies métaboliques et/ou de signalisations sous-jacentes à la prolifération et/ou la différenciation des cellules de l'épiderme.

Il existe encore un besoin de disposer de nouveaux outils de criblage de composés actifs aptes à moduler la prolifération et/ou la différenciation des cellules de l'épiderme.

Il existe encore un besoin de disposer de nouveaux composés actifs aptes à modifier, corriger, restaurer et/ou renforcer les fonctions physiologiques de l'épiderme.

Il existe encore un besoin de disposer de nouveaux composés actifs aptes à exercer un effet de soin cosmétique ou thérapeutique à l'égard de la peau ou de ses annexes.

Il existe encore un besoin de disposer de nouveaux composés actifs aptes à prévenir et/ou traiter un défaut esthétique ou un trouble pathologique de la peau ou de ses annexes, résultant d'un défaut de prolifération et/ou de différenciation des cellules de l'épiderme.

La présente invention a pour objet de satisfaire ces besoins.

Ainsi, la présente invention concerne une utilisation cosmétique non thérapeutique d'au moins un composé apte à moduler l'interaction entre une première et une seconde protéines partenaires, des homologues ou des fragments desdites protéines, lesdites première et seconde protéines étant la SASPase et la Filaggrine-2, ou FLG2, à titre d'agent actif pour traiter et/ou prévenir un défaut esthétique de la peau et/ou de ses annexes lié à un déséquilibre de la différentiation et/ou de la prolifération des cellules d'un épiderme,
ledit composé étant représenté par les formules générales (Ia) ou (Ib) suivantes : 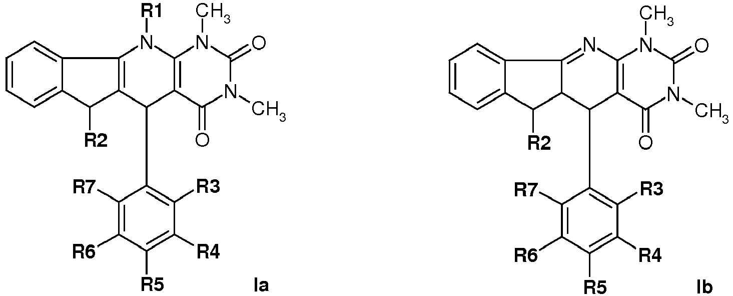 dans lesquelles :
- R¹ représente H, un alkyle en C₁-C₄, linéaire ou ramifié, saturé ou insaturé, ou -C(O)R⁸, dans lequel R⁸ représente un alkyle en C₁-C₄, linéaire ou ramifié, saturé ou insaturé, R¹ représentant de préférence H, un méthyle, un éthyle, un propyle, ou -C(O)R⁸ dans lequel R⁸ représente un méthyle, un éthyle ou un propyle ;
- R² représente =O, -OR⁹ ou -OC(O)R⁹, dans lesquels R⁹ représente H ou un alkyle saturé en C₁-C₂, R² représentant de préférence =O ou -OR⁹, préférentiellement =O ;
- R³, R⁴, R⁵, R⁶, R⁷ représentent, indépendamment l'un de l'autre, H ; -NO₂ ; -OH ; un fluor ; -CF₃ ; un alkyle en C₁-C₄, linéaire ou ramifié, saturé ou insaturé ; un phényle ; -OC(O)-CH(Ph)₂ ; -O-Ph-X avec X représentant H, -OH, -NO₂, un fluor, un alkyle ou un alkoxy en C₁-C₄, linéaire ou ramifié, saturé ou insaturé ; -O-S(O₂)-Ph-Z avec Z représentant un alkyle en C₁-C₄, linéaire ou ramifié, saturé ou insaturé ; -R⁸OH ; -OR⁹ ; -OC(O)R⁹ avec R⁸ et R⁹ étant tels que définis précédemment ; -R¹⁰Ph avec R¹⁰ étant un alkylène en C₁-C₄, linéaire ou ramifié, saturé ou insaturé ; ou -OC(O)-Yₙ-Ar₁-(X)ₘ avec
- n figurant 0 ou 1 et m figurant un entier variant de 0 à 4, sous réserve que n et m ne figurent pas simultanément 0, Y représentant un alkylène en C₁-C₄, linéaire ou ramifié, saturé ou insaturé, X étant tel que défini précédemment, et Ar₁ figurant 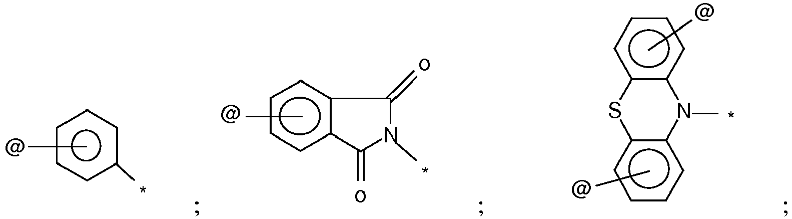 ou 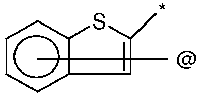 avec * figurant une liaison avec Y et @ figure une liaison avec X,
   et leurs sels physiologiquement acceptables,
   les homologues ayant une séquence présentant une identité d'au moins 85 % avec l'une des protéines partenaires et une activité biologique de même nature, et
   les fragments étant des portions d'une protéine partenaire comprenant de 6 à 260 acides aminés contigus et étant dotés d'une activité biologique de même nature.

Par la suite, les termes « protéine » ou « séquence protéique » sont utilisés de manière équivalente, et indifféremment, sauf indications contraires, pour désigner une protéine, un homologue, ou un fragment de celle-ci.

Au sens de l'invention, on entend par « moduler », le fait de favoriser, réduire ou stabiliser l'interaction entre des protéines.

De manière inattendue, les inventeurs ont mis en évidence, d'une part, la manifestation d'une interaction entre la protéase à acide aspartique SASPase et la protéine Filaggrine-2, ou FLG2, plus particulièrement via la partie N-terminale correspondant à la partie S100 de cette protéine, dans l'épiderme et, d'autre part, que cette interaction favorisait l'activité protéolytique de la SASPase.

FR 2 842 209 enseigne que la protéine SASPase est fortement exprimée dans l'épiderme, et est impliquée dans les processus de desquamation de la peau, et décrit cette protéine comme cible dans le traitement de désordres cutanés. Toutefois, ce document ne fait pas mention de l'interaction SASPase-FLG2, et de l'implication de ce complexe protéique dans la régulation de la différentiation ou la prolifération des cellules de l'épiderme.

En outre, les inventeurs ont montré que l'interaction SASPase-FLG2 pouvait être modulée au moyen de différents composés actifs, et que cette modulation pouvait être utile pour prévenir et/ou traiter un défaut esthétique ou un trouble pathologique de la peau et/ou de ses annexes, consécutive à un déséquilibre de la différenciation et/ou de la prolifération des cellules de l'épiderme.

Au sens de l'invention, on entend par « réguler la prolifération et/ou la différenciation des cellules d'un épiderme », le fait de rétablir un équilibre physiologique entre ces deux processus.

Au sens de la présente invention, on entend par « prévenir » le fait de réduire le risque ou la probabilité de manifestation d'un phénomène donné, i.e. dans la présente invention, un défaut esthétique ou un trouble pathologique de la peau et/ou de ses annexes lié à un déséquilibre de la différenciation et/ou de la prolifération des cellules de l'épiderme.

Selon un autre aspect, la présente invention concerne l'utilisation cosmétique d'au moins un composé tel que défini précédemment, à titre d'agent actif pour favoriser la ré-épithélialisation après un traitement de gommage cutané ou exfoliant.

Selon encore un autre aspect, la présente invention concerne l'utilisation cosmétique non thérapeutique d'au moins un composé tel que défini ci-dessus, à titre d'agent actif pour prévenir et/ou traiter la chute des cheveux et/ou favoriser la repousse des cheveux, ou pour prévenir la repousse des poils.

Selon un autre mode de réalisation, la présente invention concerne l'utilisation *in vitro* ou *ex vivo* d'au moins un complexe protéique comprenant une première et une seconde protéines partenaires interagissant l'une avec l'autre, lesdites première et seconde protéines étant la SASPase et la Filaggrine-2, ou FLG2, des homologues ou des fragments desdites protéines, pour cribler des composés aptes à réguler la prolifération et/ou la différenciation des cellules d'un épiderme par modulation de l'interaction entre lesdites première et seconde protéines,
les homologues ayant une séquence présentant une identité d'au moins 85 % avec l'une des protéines partenaires et une activité biologique de même nature, et
les fragments étant des portions d'une protéine partenaire comprenant de 6 à 260 acides aminés contigus et étant dotés d'une activité biologique de même nature.

Au sens de l'invention, on entend par « réguler la prolifération et/ou la différenciation des cellules d'un épiderme », le fait de rétablir un équilibre physiologique entre ces deux processus.

Selon un autre mode de réalisation, la présente invention concerne un procédé de criblage *in vitro* ou *ex vivo* d'un composé apte à réguler la prolifération et/ou la différenciation des cellules d'un épiderme par modulation de l'interaction entre une première et une seconde protéines partenaires, des homologues ou des fragments desdites protéines, lesdites première et seconde protéines étant la SASPase et la Filaggrine-2, ou FLG2, ledit procédé comprenant au moins les étapes décrites ci-après.

Selon un autre mode de réalisation, la présente invention concerne une composition pharmaceutique ou dermatologique comprenant dans un milieu physiologiquement acceptable au moins une quantité efficace d'au moins un composé de l'invention, pour prévenir et/ou traiter un désordre pathologique de la peau et/ou de ses annexes lié à un déséquilibre de la différentiation et/ou de la prolifération des cellules d'un épiderme.

Selon un autre mode de réalisation, la présente invention concerne une composition cosmétique comprenant à titre d'agent actif, dans un milieu physiologiquement acceptable, au moins une quantité efficace d'au moins un composé de l'invention, et au moins un ingrédient cosmétiquement ou dermatologiquement acceptable choisi parmi des actifs cosmétiques ou dermatologiques, des conservateurs, des gélifiants hydrophiles ou lipophiles, des agents tensioactifs non ioniques, anioniques, cationiques, des antioxydants, des sels, des parfums, des charges, des absorbeurs d'odeur, des matières colorantes, des polymères filmogènes, des polymères semi-cristallins, des gommes, des huiles volatiles ou non volatiles, et leurs mélanges.

Au sens de la présente invention, on entend par « milieu physiologiquement acceptable », un milieu compatible avec une administration à un individu, notamment sur ou dans la peau et/ou de ses annexes de cet individu.

Au sens de la présente invention, on entend par « quantité efficace » d'un composé de l'invention, une quantité suffisante et nécessaire de ce composé pour exercer un effet de prévention et/ou de traitement à l'égard d'un défaut esthétique ou d'un désordre pathologique de la peau et/ou de ses annexes. Une telle quantité peut être déterminée par toute méthode connue de l'homme de l'art, par exemple au moyen d'essais *in vitro, ex vivo* ou *in vivo,* tels que des essais cliniques.

Selon encore un autre aspect, la présente invention concerne un article de conditionnement d'une composition cosmétique, pharmaceutique ou dermatologique, contenant au moins une composition cosmétique, pharmaceutique ou dermatologique comprenant au moins un composé de l'invention.

Selon un autre mode de réalisation, la présente invention concerne un procédé cosmétique pour prévenir et/ou traiter un défaut esthétique de la peau et/ou de ses annexes lié à un déséquilibre de la différentiation et/ou de la prolifération des cellules de l'épiderme chez un individu, comprenant au moins une étape d'administration audit individu d'au moins un composé de l'invention.

Selon un autre mode de réalisation, la présente invention concerne un complexe protéique comprenant une première et une seconde protéines partenaires interagissant l'une avec l'autre, lesdites première et seconde protéines étant la SASPase et la Filaggrine-2, ou FLG2, des homologues ou des fragments desdites protéines,
les homologues ayant une séquence présentant une identité d'au moins 85 % avec l'une des protéines partenaires et une activité biologique de même nature, et
les fragments étant des portions d'une protéine partenaire comprenant de 6 à 260 acides aminés contigus et étant dotés d'une activité biologique de même nature.

La présente invention permet avantageusement de disposer d'une nouvelle cible utilisable en cosmétique ou en thérapeutique à l'égard de la peau et/ou de ses annexes, et notamment vis-à-vis de la peau.

Avantageusement encore, la présente invention permet de fournir un nouvel outil de criblage particulièrement efficace et sensible pour la détection de nouveaux agents actifs utiles en cosmétique ou en thérapeutique à l'égard de la peau et/ou de ses annexes.

La présente invention permet avantageusement de disposer de nouveaux agents actifs utiles en cosmétique ou en thérapeutique, et aptes à réguler la prolifération et/ou la différenciation des cellules de l'épiderme, et qui soient en particulier dotés d'une bonne efficacité et d'une bonne tolérance.

La présente invention permet avantageusement de disposer de nouvelles compositions cosmétiques ou thérapeutiques particulièrement efficaces à l'égard de défauts esthétiques ou de troubles pathologiques de la peau et/ou de ses annexes liés à un déséquilibre de la prolifération et/ou de la différenciation des cellules de l'épiderme.

Enfin, la présente invention permet avantageusement de disposer d'un nouveau procédé cosmétique de traitement ou de prévention particulièrement efficace à l'égard de défauts esthétiques de la peau et/ou de ses annexes liés à un déséquilibre de la prolifération et/ou de la différenciation des cellules de l'épiderme.

### Complexe SASPase-FLG2

Comme indiqué précédemment, l'invention concerne un complexe protéique résultant de l'interaction entre deux protéines, la SASPase et la FLG2, et l'utilisation de ce complexe comme marqueur et cible biologique de la différentiation et/ou de la prolifération des cellules d'un épiderme.

Un complexe de l'invention peut être formé par interaction de tout ou partie d'une première séquence protéique de la SASPase avec tout ou partie d'une seconde séquence protéique de la FLG2 ou un dimère de cette seconde séquence.

La SASPase ou « Skin Aspartic Protease », est une protéine de la famille des protéases dites à acide aspartique, comprenant 343 acides aminés (Uniprot/Swissprot : Q53RT3) et présentant un poids moléculaire d'environ 37 kDa.

Sa forme courte comprend 259 acides aminés, pour un poids moléculaire d'environ 28 kDa, et est représentée par une séquence s'étendant de la position 85 à la position 343 de la séquence Q53RT3 (Référence Uniprot/Swissprot). De manière préférée, une séquence protéique de la SASPase utile pour l'invention est représentée par la séquence s'étendant de la position 85 à la position 343 de la séquence Q53RT3.

La Filaggrine-2, ou FLG2, est une protéine de 2391 acides aminés présentant un poids moléculaire d'environ 248 kDa (Réf. Uniprot/Swissprot : Q5D862).

La FLG2 est une protéine de type « fused-type » S100, de structure proche de celle de la Filaggrine, exprimée dans l'épiderme, et présentée comme étant susceptible d'être impliquée dans la différenciation des cellules de l'épiderme, la formation de la barrière épidermique, la protection de la peau face aux agressions de l'environnement, l'organisation du réseau de filaments intermédiaires de kératines conférant sa résistance à l'épiderme, ainsi que dans l'hydratation cutanée (Wu et al., PLoS One, 2009 ; 4(4):e5227).

Selon un mode de réalisation, la FLG2 mise en oeuvre selon l'invention peut être représentée par une séquence d'acides aminés s'étendant des positions 2 à 213 de la séquence Q5D862, et plus préférentiellement des positions 2 à 95, ou un dimère de ces séquences.

La mise en oeuvre de l'invention s'étend également aux séquences d'acides nucléiques codant pour les protéines, les homologues, et les fragments des protéines convenant à l'invention.

Par « homologue » d'une séquence protéique ou d'une séquence d'acides nucléiques, on entend désigner une séquence présentant une identité d'au moins 85 %, en particulier d'au moins 90 %, en particulier d'au moins 98 %, et plus particulièrement d'au moins 99 % avec ladite séquence, et possédant une activité biologique de même nature.

Une homologie de séquence peut être identifiée par toute technique habituellement utilisée dans le domaine par l'homme de l'art. A titre d'exemple, une homologie de séquence peut être déterminée par utilisation de l'interface informatique BLAST disponible sur le site Internet NCBI à l'adresse http://blast.ncbi.nlm.nih.gov configurée avec les paramètres par défaut.

Un homologue d'une séquence protéique peut différer de cette séquence, par exemple, par une ou plusieurs délétion(s), et/ou insertion(s) et/ou une ou plusieurs substitution(s) d'un acide aminé. De manière similaire, un homologue d'une séquence d'acides nucléiques peut différer de cette séquence, par exemple, par une ou plusieurs délétion(s), et/ou insertion(s) et/ou une ou plusieurs substitution(s) d'une base ou d'un codon. Ces modifications peuvent être combinées.

Selon une variante de réalisation, un homologue d'une séquence protéique ou d'acides nucléiques peut comprendre une ou plusieurs substitutions conservatrices d'acides aminés ou de codons.

Une substitution conservatrice est le remplacement, dans une séquence protéique, d'un acide aminé par un autre acide aminé doté de propriétés physico-chimiques sensiblement similaires, ou suffisamment proches, de celles de l'acide aminé d'origine, pour que les propriétés et fonctions de la protéine ne soient pas, ou sensiblement pas, affectées. De même, une substitution conservatrice est le remplacement, dans une séquence d'acides nucléiques, d'un codon par un autre codon codant pour un acide aminé identique ou doté de propriétés physico-chimiques sensiblement similaires, ou suffisamment proches, de celles de l'acide aminé d'origine pour que les propriétés et fonctions de la séquence d'acides nucléiques codée ne soient pas ou sensiblement pas, affectées.

Les modifications des séquences protéiques ou d'acides nucléiques présentées ci-dessus peuvent être dénommées, de manière générale, « mutation ». Ainsi, les homologues de l'invention concernent également les variants des séquences d'acides aminés ou d'acides nucléiques de l'invention.

A titre d'exemple de variants que l'on peut considérer dans la présente invention, on peut mentionner le remplacement d'un ou plusieurs résidus d'acides aminés par des résidus d'acides aminés ayant un indice hydropathique similaire, sans pour autant affecter de manière sensible les propriétés biologiques de la protéine, et notamment sa propriété d'interaction avec sa protéine partenaire. L'indice hydropathique est un indice attribué aux acides aminés en fonction de leur hydrophobicité et de leur charge (Kyte et Doolittle (1982), J. Mol. Biol., 157 : 105).

Une protéine de l'invention peut comprendre une ou plusieurs modification(s) post-traductionnelle(s).

Par « modification(s) post-traductionnelle(s) », on entend englober l'ensemble des modifications qu'un peptide ou une protéine est susceptible de subir à l'issu de sa synthèse dans une cellule, telle(s) que par exemple une ou des phosphorylation(s), une ou des glycosylation(s), une ou des citrullination(s), une ou des lipidation(s), telles qu'une farnésylation ou une palmitoylation, un réarrangement structural de type formation de ponts disulfures et/ou clivage à l'intérieur de la séquence protéique.

Une homologie de séquence peut être appréciée à l'égard de l'ensemble de la séquence de la protéine ou d'une ou plusieurs séquences caractéristiques de cette protéine.

On entend par « séquence caractéristique » d'une protéine, une séquence d'acides aminés déterminante dans l'identité de la protéine et impliquée dans son activité biologique, comme par exemple le maintien de sa structure tridimensionnelle, son affinité avec les éléments cellulaires avec lesquels la protéine interagit habituellement, comme d'autres protéines, des séquences d'acides nucléiques, des sucres ou des lipides, son activité enzymatique ou son activité de substrat à l'égard d'autres enzymes.

Plus particulièrement, des séquences caractéristiques de la SASPase ou de la FLG2 convenant à l'invention sont des séquences impliquées dans l'interaction de ces protéines l'une avec l'autre.

Selon un mode de réalisation, les séquences caractéristiques plus particulièrement considérées peuvent être les séquences N-terminal des protéines SASPase et FLG2.

Selon une variante de réalisation particulière, une séquence caractéristique de la SASPase convenant à l'invention peut être comprise dans la partie N-terminale de la SASPase, forme courte et plus particulièrement peut être une séquence s'étendant de la position 97 à la position 173 de la séquence Q53RT3 (Référence Uniprot/Swissprot).

Selon une autre variante de réalisation, une séquence caractéristique de la FLG2 convenant à l'invention peut être comprise dans la partie N-terminale de la FLG2, et plus particulièrement peut être une séquence s'étendant de la position 2 à la position 95 de la séquence Q5D862 (référence Uniprot/Swissprot).

De manière préférée, les séquences caractéristiques de la SASPase et/ou de la FLG2 peuvent avantageusement être mises en oeuvre dans l'invention.

Par « activité biologique de même nature » d'une protéine de l'invention, on entend sa capacité à interagir avec la seconde protéine partenaire de l'invention. Par « activité biologique de même nature » d'une séquence d'acides nucléiques, on entend en particulier sa capacité à être transcrite et traduite en une séquence d'acides aminés.

A l'égard de la SASPase, une activité biologique de même nature peut s'entendre également de son activité protéolytique, notamment à l'égard de la cornéodesmosine.

A l'égard de la FLG2, une activité biologique de même nature peut également s'entendre de sa propriété à interagir avec la SASPase et/ou à favoriser et augmenter l'activité protéolytique de la SASPase.

Selon une autre variante de l'invention, une activité biologique de même nature peut également s'entendre de l'activité biologique d'un complexe de l'invention à l'égard de la prolifération et/ou de la différenciation des cellules de l'épiderme.

Ainsi, un homologue ou un fragment des première et seconde protéines partenaires convenant à l'invention sont nécessairement dotés au moins de la propriété d'interagir l'un avec l'autre pour former un complexe convenant à l'invention.

Au sens de l'invention, on entend par « fragment d'une protéine » toute portion d'une protéine conforme à l'invention comprenant de 6 à 260 acides aminés, de préférence de 3 à 215 acides aminés, de préférence de 10 à 180 acides aminés, et de préférence encore de 20 à 140, voire de 40 à 100, ou encore de 60 à 100 acides aminés contigus, et dotée d'une activité biologique de même nature que celle exprimée par la protéine.

Le terme « fragment » à l'égard d'une séquence d'acides nucléiques désigne toute portion de cette séquence, et constituée de 18 à 780 bases, de préférence de 24 à 645 bases, de préférence de 30 à 540 bases, et de préférence encore de 6 à 420 bases, voire de 120 à 300 ou encore de 180 à 300 bases, et codant pour un fragment de la séquence d'acides aminés codée ladite séquence.

De préférence, un fragment de l'invention est issu de la partie N-terminale d'une protéine de l'invention.

Selon une variante de réalisation, une protéine SASPase convenant à l'invention peut comprendre uniquement une séquence d'acides aminés s'étendant de la position 95 à 160 de la protéine SASPase (référence Uniprot/Swissprot : Q53RT3).

Une telle séquence conserve la propriété d'interaction de la SASPase avec la FLG2, mais est dénuée d'activité protéolytique.

Selon un autre mode de réalisation, la FLG2 mise en oeuvre selon l'invention peut consister en les 100 premiers acides aminés N-terminaux, voire en les 95 premiers acides aminés N-terminaux, et de manière préférentielle peut consister en une séquence d'acides aminés s'étendant des positions 2 à 95 de la séquence Q5D862 (Uniprot/Swissprot).

Selon un mode de réalisation, une protéine convenant à l'invention peut être une protéine naturelle ou synthétique, le cas échéant susceptible d'être obtenue par synthèse chimique ou biologique, ou par extraction à partir d'un tissu biologique, comme par exemple la peau, exprimant naturellement ou après transduction une protéine, ainsi que les différentes formes post-traductionnelles de celle-ci, ainsi que les homologues, ou les fragments de celle-ci.

Selon un autre mode de réalisation, l'invention concerne une protéine chimère comprenant une première séquence protéique fusionnée ou couplée avec une seconde séquence protéique, un agent de ciblage hydrophile ou hydrophobe, un précurseur de bioconversion, un marqueur d'affinité par exemple un marqueur fluorescent, un marqueur luminescent, un marqueur radioactif ou un marqueur colorimétrique.

De manière non limitative, on peut citer comme exemple de composés susceptibles d'être couplés avec une protéine conforme à l'invention, des protéines fluorescentes comme la « Green Fluorescent Protein », des composés chimiques fluorescents tels que la rhodamine, la fluorescéine, ou le Texas Red, des composés phosphorescents, des éléments radioactifs, tels que ³H, ³⁵S, ¹²¹I ou ¹²⁵I, des marqueurs colorimétriques comme les substrats chromogènes sensibles à l'action de la galactosidase, de la peroxydase, de la chloramphénicol acétyltransférase, de la luciférase ou de la phosphatase alcaline, ou encore des marqueurs d'affinité.

Selon un mode préféré de réalisation, une protéine selon l'invention peut être couplée à un marqueur d'affinité, en particulier choisi parmi un marqueur Myc, FLAF, His, His-tag, GST (Glutathion S-Transférase), THX (Thiorédoxine), MBP (Maltose-Binding Protein), THX-His-tag ou THX-His-S-tag.

Selon la nature des composés susceptibles d'être couplés avec une protéine conforme à l'invention, le couplage peut être opéré par tout procédé chimique ou tout procédé de biologie moléculaire connu de l'homme de l'art. Dans le dernier cas, on peut parler de protéine de fusion ou de protéine chimère.

Ainsi, selon un mode de réalisation, une protéine SASPase convenant à l'invention peut en particulier être une protéine recombinante fusionnée à un marqueur d'affinité.

Selon un mode de réalisation, une protéine FLG2 recombinante convenant à l'invention peut être une protéine recombinante fusionnée à un marqueur d'affinité.

Une protéine recombinante de l'invention, , tronquée ou non, le cas échéant fusionnée à une autre protéine, par exemple un marqueur d'affinité, peut être obtenue par tout procédé de biologie moléculaire, notamment comme décrit par SAMBROOK et al. (Molecular Cloning : A laboratory Manual, Ed. Cold Spring Harbor, 2001).

La mise en oeuvre de l'invention s'étend également aux séquences d'acides nucléiques codant pour les protéines, les homologues, et les fragments des protéines convenant à l'invention.

Une séquence d'acides nucléiques codant pour la protéine SASPase et convenant à l'invention peut être, par exemple, la séquence d'acides nucléiques identifiée sous la référence GenBank AK055994, AK057813, BC031997, BC094000 ou BC094002, de préférence sous la référence GenBank AK055994.

Une séquence d'acides nucléiques codant pour une protéine FLG2 et convenant à l'invention peut être, par exemple, la séquence d'acides nucléiques identifiée sous la référence GenBank AY827490.1.

Une séquence d'acide nucléique convenant à l'invention peut en particulier être mise en oeuvre pour la préparation de toutes protéines conformes à l'invention, et plus particulièrement pour la préparation de protéines fusionnées à un marqueur d'affinité.

Une séquence d'acides nucléiques convenant à l'invention peut être issue de toute origine possible, à savoir soit animale, en particulier de mammifères et encore plus particulièrement humaine, soit végétale, soit de micro-organismes, tels que des virus, des phages, des bactéries ou encore des champignons, sans préjuger du fait qu'elle soit présente de manière naturelle ou non dans ledit organisme.

### Composés modulateurs

Au sens de l'invention, on entend par « composé modulateur » tout composé apte à agir directement sur un complexe protéique de l'invention en vue de moduler l'interaction entre les protéines partenaires de ce complexe.

Au sens de l'invention, on entend par « moduler » le fait de favoriser, réduire ou stabiliser l'interaction entre ces protéines.

Un composé modulateur de l'invention peut se lier à une ou aux deux protéines partenaires. Cette liaison peut s'effectuer, par exemple, au niveau du ou des domaines des protéines impliquées dans l'interaction. Ainsi, un composé modulateur de l'invention peut interagir plus particulièrement avec un domaine de la partie N-terminale de la forme courte de la SASPase, ou de la partie N-terminale de la FLG2. Alternativement, un composé modulateur peut se lier simultanément au domaine des séquences protéiques de la SASPase et de la FLG2 impliquées dans la formation de complexes.

Alternativement, cette liaison peut s'effectuer à distance du site d'interaction des protéines, conduisant par allostérie à un changement conformationnel de l'une ou des protéines, et à une modulation de l'interaction entre les deux protéines, ou de la fonction de ces protéines.

Selon le composé modulateur considéré, l'interaction entre la SASPase et la FLG2 peut être renforcée, réduite, supprimée ou stabilisée.

Selon un mode de réalisation, un composé modulateur peut favoriser ou être agoniste de l'interaction SASPase-FLG2. Alternativement, un composé modulateur peut être un composé déstabilisant ou antagoniste de l'interaction SASPase-FLG2.

La nature agoniste ou antagoniste d'un composé modulateur de l'interaction SASPase-FLG2 sera retenue selon l'effet modulateur à exercer sur la prolifération et/ou la différenciation des cellules de l'épiderme, et plus particulièrement selon la nature du défaut esthétique ou du trouble pathologique de la peau ou de ses annexes à prévenir ou à traiter.

Plus particulièrement, dans le cadre de défauts esthétiques ou de troubles pathologiques liés à une insuffisance de la desquamation ou à une accélération de la prolifération et/ou de la différenciation des cellules de l'épiderme, on pourra préférer un composé modulateur agoniste de, ou favorisant, l'interaction SASPase-FLG2.

Un tel composé pourra exercer une activité pro-desquamante et/ou anti-âge des cellules de l'épiderme, favorisant ainsi l'épaississement de l'épiderme vivant et régulant l'épaisseur du *stratum corneum.*

Alternativement, à l'égard de défauts esthétiques ou de troubles pathologiques de la peau et/ou de ses annexes résultant d'une desquamation excessive ou d'une insuffisance de la prolifération et/ou de la différenciation des cellules de l'épiderme, on pourra mettre avantageusement en oeuvre un composé modulateur antagoniste de, ou déstabilisant, l'interaction SASPase-FLG2.

Un tel composé pourra exercer une activité pro-différentiante des cellules de l'épiderme favorisant la maturation de la fonction barrière.

Le composé considéré selon l'invention peut être représenté par les formules générales (Ia) ou (Ib) suivantes : 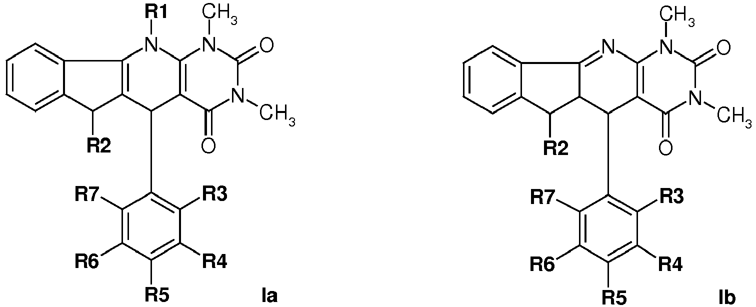 dans lesquelles :
- R¹ représente H, un alkyle en C₁-C₄, linéaire ou ramifié, saturé ou insaturé, ou -C(O)R⁸, dans lequel R⁸ représente un alkyle en C₁-C₄, linéaire ou ramifié, saturé ou insaturé ;
- R² représente =O, -OR⁹ ou -OC(O)R⁹, dans lesquels R⁹ représente H ou un alkyle en C₁-C₂ ;
- R³, R⁴, R⁵, R⁶, R⁷ représentent, indépendamment l'un de l'autre, H ; -NO₂ ; -OH ; un fluor ; -CF₃ ; un alkyle en C₁-C₄, linéaire ou ramifié, saturé ou insaturé ; un phényle ; -OC(O)-CH(Ph)₂ ; -O-Ph-X avec X représentant H, -OH, -NO₂, un fluor, un alkyle ou un alkoxy en C₁-C₄, linéaire ou ramifié, saturé ou insaturé ; -O-S(O₂)-Ph-Z avec Z représentant un alkyle en C₁-C₄, linéaire ou ramifié, saturé ou insaturé ; -R⁸OH ; -OR⁹ ; -OC(O)R⁹ avec R⁸ et R⁹ étant tels que définis précédemment ; -R¹⁰Ph avec R¹⁰ étant un alkylène en C₁-C₄, linéaire ou ramifié, saturé ou insaturé ; ou -OC(O)-Yₙ-Ar₁-(X)ₘ avec
- n figurant 0 ou 1 et m figurant un entier variant de 0 à 4, sous réserve que n et m ne figurent pas simultanément 0, Y représentant un alkylène en C₁-C₄, linéaire ou ramifié, saturé ou insaturé, X étant tel que défini précédemment, et Ar₁ figurant 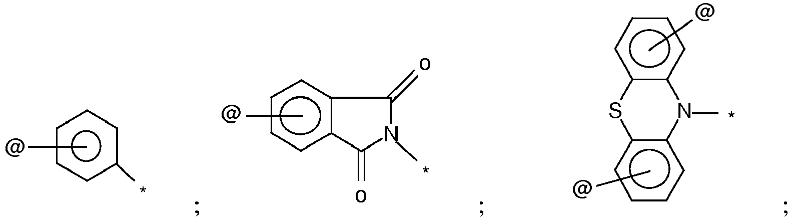 ou 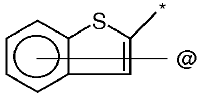 avec * figurant une liaison avec Y et @ figure une liaison avec X et leurs sels physiologiquement acceptables.

R¹ peut, notamment, représenter H, un alkyle en C₁-C₃ linéaire ou ramifié, saturé ou insaturé, ou -C(O)R⁸ dans lequel R⁸ représente un alkyle en C₁-C₃, linéaire ou ramifié, saturé ou insaturé.

De manière préférée, R¹ peut représenter H, un méthyle, un éthyle, un propyle, ou -C(O)R⁸ dans lequel R⁸ représente un méthyle, un éthyle ou un propyle.

De manière encore plus préférée, R¹ peut représenter un méthyle, un éthyle ou un propyle, et de préférence H.

En particulier, un composé de l'invention peut être représenté par la formule (Ia) dans laquelle R¹ représente H, un méthyle, un éthyle, ou un propyle, et de préférence représente H.

R² peut représenter =O ou -OR⁹, R⁹ étant tél que défini précédemment.

Avantageusement, R² peut représenter =O ou -OR⁹ dans lesquels R⁹ représente H ou un méthyle.

De manière préférée, R² peut représenter =O.

Selon un mode de réalisation, R³, R⁴, R⁵, R⁶ et R⁷ peuvent représenter, indépendamment l'un de l'autre, H ; -OH ; un fluor ; un alkyle en C₁-C₃, linéaire ou ramifié, saturé ou insaturé ; un phényle ; -OC(O)-CH(Ph)₂ ; -O-Ph-X avec X représentant H, -OH, -NO₂, un fluor, un alkyle ou un alkoxy en C₁-C₃, linéaire ou ramifié, saturé ou insaturé ; -O-S(O₂)-Ph-Z avec Z représentant un alkyle en C₁-C₃, linéaire ou ramifié, saturé ou insaturé ;-R⁸OH ; -OR⁹ ; -OC(O)R⁹ avec R⁸ représentant un alkyle en C₁-C₃, linéaire ou ramifié, saturé ou insaturé, et R⁹ représentant H ou un méthyle ; -R¹⁰Ph avec R¹⁰ étant un alkylène en C₁-C₃ linéaire ou ramifié, saturé ou insaturé ; ou -OC(O)-Yₙ-Ar₁-(X)ₘ avec
- n figurant 0 ou 1 et m figurant un entier variant de 0 à 4, Y représentant un alkylène en C₁-C₃, linéaire ou ramifié, saturé ou insaturé, X représentant H, -OH, un fluor, un alkyle ou un alkoxy en C₁-C₃, linéaire ou ramifié, saturé ou insaturé, Ar₁ figurant : 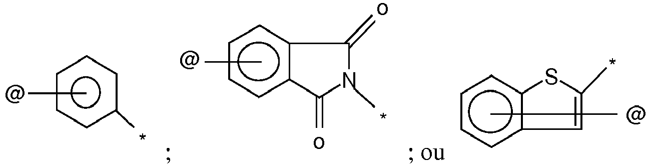 avec * figurant une liaison avec Y et @ figure une liaison avec X.

Selon un mode de réalisation, R³, R⁴, R⁵, R⁶ et R⁷ peuvent représenter, indépendamment l'un de l'autre, H ; -OH ; un fluor ; un méthyle ou un éthyle ; un phényle ; -OC(O)-CH(Ph)₂ ; -O-Ph-X avec X représentant H, -OH, un fluor, un méthyle, un éthyle, un méthoxy ou un éthoxy ; -O-S(O₂)-Ph-Z avec Z représentant un méthyle ou un éthyle ; -R⁸OH ; -OR⁹ ; -OC(O)R⁹ avec R⁸ représentant un méthyle ou un éthyle et R⁹ représentant H ou un méthyle ; -R¹⁰Ph avec R¹⁰ étant un méthylène ou un éthylène, ou -OC(O)-Yₙ-Ar₁-(X)ₘ avec n, Y, X et Ar₁ étant tels que définis précédemment.

Selon un mode de réalisation, R³, R⁴, R⁵, R⁶ et R⁷ peuvent représenter, indépendamment l'un de l'autre, H ; -OH ; un fluor ; un méthyle ou un éthyle ; - R⁸OH avec R⁸ représentant un méthyle ou un éthyle ; -OR⁹ avec R⁹ représentant H ou un méthyle ; ou -OC(O)-Yₙ-Ar₁-(X)ₙ avec n, Y, X et Ar₁ étant tels que définis précédemment.

Selon un mode de réalisation, un de R³, R⁴, R⁵, R⁶ et R⁷ peut représenter -OC(O)-Yn-Ar₁-X)ₘ avec n figurant 0 ou 1 et m figurant un entier variant de 0 à 3, et notamment de 1 à 2, Y représentant un alkylène en C₁-C₃, linéaire ou ramifié, saturé ou insaturé, et notamment un méthylène ou un éthylène, X peut représenter H, -OH, un méthyle, un éthyle, un méthoxy ou un éthoxy, notamment H, un méthyle ou un méthoxy, et de préférence un éthyle ou un éthoxy, et Ar₁ figurant :

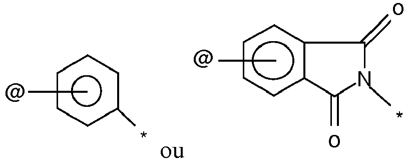

Selon un mode de réalisation préféré, dans le groupe -OC(O)-Yₙ-Ar₁-Xₘ, Ar₁ peut être : 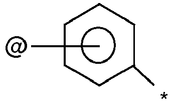 dans lequel * et @ sont tels que définis précédemment.

Selon un autre mode de réalisation préféré, dans le groupe -OC(O)-Yₙ-Ar₁-Xₘ, Y peut être choisi parmi un méthylène, un éthylène, un propylène, un isopropylène, et plus préférentiellement peut être choisi parmi un méthylène et un isopropylène.

Selon un autre mode de réalisation préféré, dans le groupe -OC(O)-Yₙ-Ar₁-Xₘ, n peut être égale à 0 et m peut être égale à 1.

Selon un autre mode de réalisation préféré, dans le groupe -OC(O)-Yₙ-Ar₁-Xₘ, X peut être choisi parmi un alkyl ou un alkoxy en C₁-C₄, de préférence en C₂-C₃, linéaire ou ramifié, saturé ou insaturé. De manière préférée, X peut être choisi parmi un méthyle, un éthyle, un méthoxy ou un éthoxy. Encore plus préférentiellement, X peut être un méthyle ou un méthoxy, et de préférence un méthyle.

Selon un mode de réalisation préféré, dans le groupe -OC(O)-Yₙ-Ar₁-Xₘ, n peut être égale à 0, m peut être égale à 1, X peut être un méthyle ou un méthoxy, de préférence un méthyle et Ar₁ peut être : 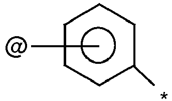 dans lequel * et @ sont tels que définis précédemment.

Selon un mode de réalisation, au plus un de R³, R⁴, R⁵, R⁶ et R⁷ peut représenter -OC(O)-Yₙ-Ar₁-Xₘ tel que défini précédemment, et de préférence R⁴ ou R⁶ représentent -OC(O)-Yₙ-Ar₁-Xₘ tel que défini précédemment. Selon une variante de ce mode de réalisation, -OC(O)-Yₙ-Ar₁-Xₘ peut être tel que n peut être égal à 0, m égal à 1, X tel que défini précédemment, et Ar₁ peut représenter 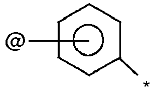 avec * figurant une liaison avec Y et @ figure une liaison avec X.

Selon un mode de réalisation, R³, R⁵, R⁷ et R⁴ ou R⁶, peuvent représenter, indépendamment l'un de l'autre, H ; -NO₂ ; -OH ; un fluor ; un méthyle ou un éthyle ; un phényle; -R⁸OH ; -OR⁹ ; -OC(O)R⁹ avec R⁸ représentant un méthyle ou un éthyle et R⁹ représentant H ou un méthyle ; -R¹⁰Ph avec R¹⁰ étant un méthylène ou un éthylène.

Selon une variante de réalisation préférée, R³, R⁵, R⁷ et R⁴ ou R⁶, peuvent représenter, indépendamment l'un de l'autre, H, -OH, un fluor, un méthyle, et de préférence représentent H.

Selon un mode de réalisation, un composé de l'invention peut être en particulier représenté par la formule générale (Ia).

Selon un mode préféré de l'invention, un composé convenant à l'invention peut être de préférence représenté par le composé de formule (II) suivante:

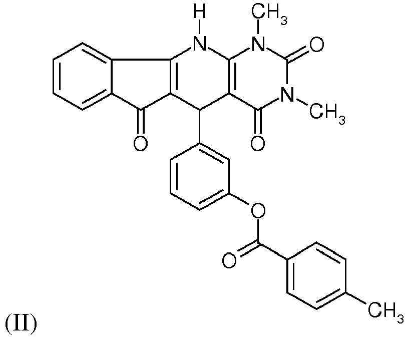

Ce composé est désigné ci-après « M7 ».

Un sel physiologiquement acceptable d'un composé de formule générale (Ia) ou (Ib) selon l'invention peut être un sel d'un composé de formule générale (Ia) ou (Ib) et d'un métal alcalin, d'un métal alcalino-terreux, ou d'ammonium, comprenant les sels obtenus avec des bases organique d'ammonium, ou des sels d'un composé de formule générale (Ia) ou (Ib) et d'acide organique ou inorganique.

Des sels convenant plus particulièrement à l'invention peuvent être des sels de sodium, de potassium, de calcium, de magnésium, des sels d'ammonium quaternaire tels que tétraméthylammonium ou le tétraéthylammonium, et des sels d'addition avec l'ammoniaque et des amines organiques physiologiquement acceptables, tel que la méthylamine, la diméthylamine, la triméthylamine, l'éthylamine, la triéthylamine, l'éthanolamine or la tris-(2-hydroxyéthyl)-amine.

Des sels d'un composé de formule générale (Ia) ou (Ib) et d'acide inorganique convenant à l'invention peuvent être obtenus avec l'acide hydrochlorique, l'acide hydrobromique, l'acide sulfurique, l'acide nitrique ou l'acide phosphorique.

Des sels d'un composé de formule générale (Ia) ou (Ib) et d'acide organique convenant à l'invention peuvent être obtenus avec ou des acide carboxyliques et des acides sulfoniques tel que l'acide formique, l'acide acétique, l'acide oxalique, l'acide citrique, l'acide lactique, l'acide malique, l'acide succinique, l'acide malonique, l'acide benzoïque, l'acide maléique, l'acide fumarique, l'acide tartarique, l'acide méthanesulfonique, l'acide benzènesulfonique ou l'acide p-toluènesulfonique.

Les composés modulateurs convenant à l'invention peuvent, notamment, être obtenus comme décrit dans la demande WO 2008/008704.

Alternativement, les composés modulateurs convenant à l'invention peuvent être obtenus à partir de la banque ChemDiv Discovery Chemistry Collection Public Database (Order Number: 4477-2039), ChemDiv, Inc. San Diego, USA)

Les composés modulateurs convenant à l'invention peuvent, notamment, être obtenus comme décrit dans Tetrahedron Letters, 2005, 46(8): 1345-1348, selon les procédés schématiques suivant :

### Synthèse en phase solide

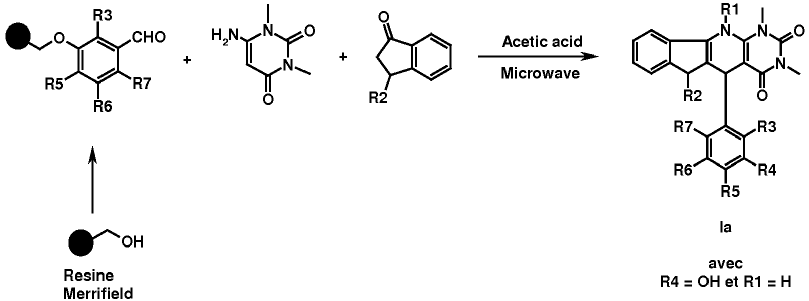

### Synthèse en phase liquide

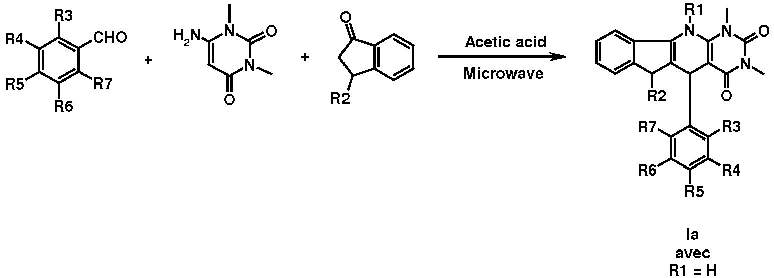

dans lesquels R¹, R², R³, R⁴, R⁵, R⁶, et R⁷ sont tels que définis ci-dessus.

### Criblage

L'invention concerne la mise en oeuvre d'un complexe protéique de l'invention pour cribler de nouveaux composés, en particulier tels que définis précédemment.

Selon un mode de réalisation, l'invention concerne un procédé de criblage d'un composé apte à réguler la prolifération et/ou la différenciation des cellules d'un épiderme par modulation de l'interaction entre une première et une seconde protéines partenaires, des homologues, ou des fragments desdites protéines, lesdites première et seconde protéines étant la SASPase et la Filaggrine-2, ou FLG2, ledit procédé comprenant au moins les étapes consistant à :
a) disposer lesdites première et seconde protéines dans des conditions propices à l'interaction entre les première et seconde protéines,
b) associer, préalablement ou postérieurement à l'étape a), au moins une des première et seconde protéines à une entité,
   ladite protéine formant avec ladite entité un ensemble apte à émettre un signal après exposition à une longueur d'onde excitatrice, et la réalisation des étapes a) et b) conduisant à l'obtention d'un complexe par interaction desdites première et seconde protéines partenaires,
c) soumettre ledit complexe à ladite longueur d'onde excitatrice pour obtenir un premier signal S1 caractéristique dudit complexe,
d) déterminer quantitativement ou qualitativement ledit signal S1,
e) mettre en contact le complexe avec un milieu présumé contenir un composé à cribler dans des conditions propices à une interaction entre ledit composé et ledit complexe,
f) soumettre le milieu obtenu à l'étape e) à ladite longueur d'onde excitatrice pour obtenir un second signal S2,
g) déterminer quantitativement ou qualitativement ledit signal S2, et
h) comparer les premier et second signaux S1 et S2 pour tirer une conclusion relative à une éventuelle modulation de l'interaction entre les première et seconde protéines par le composé criblé,
   les homologues ayant une séquence présentant une identité d'au moins 85 % avec l'une des protéines partenaires et une activité biologique de même nature, les fragments étant des portions d'une protéine partenaire comprenant de 6 à 260 acides aminés contigus et étant dotés d'une activité biologique de même nature.

Selon un mode de réalisation, une entité convenant à l'invention peut être choisie parmi un support convenant à la résonance plasmonique de surface, un marqueur fluorescent, un anticorps ou une combinaison d'anticorps primaire et secondaire, ledit anticorps ou ledit anticorps secondaire portant un marqueur fluorescent.

Une protéine de l'invention peut être associée à une entité par toute méthode connue de l'homme de l'art, et adaptée à la nature du support à laquelle la protéine doit être associée. Une méthode de couplage sera naturellement choisie de sorte à ne pas affecter les propriétés d'interaction des protéines de l'invention l'une avec l'autre.

Selon un mode de réalisation, une association entre une entité et une protéine de l'invention peut être effectuée par couplage chimique, par biologie moléculaire, ou par interaction(s) spécifique(s) de forte affinité.

En particulier, une association peut être opérée par un couplage chimique, notamment au moyen de fonctions chimiques réactives, ou par un procédé de biologie moléculaire.

Lors de la mise en oeuvre d'une association par procédé chimique, les fonctions chimiques à faire réagir peuvent être naturellement présentes sur la protéine et l'entité associée, ou peuvent être introduites sur la protéine et l'entité préalablement à la réaction chimique.

A titre d'exemple de fonctions chimiques réactives convenant à l'invention, on peut par exemple mentionner celles classiquement utilisées dans les réactions dites de « chimie click » (« click chemistry »).

A titre d'exemple de procédés de biologie moléculaire convenant à l'association entre une entité et une protéine de l'invention, on peut citer ceux classiquement mis en oeuvre dans les procédés de clonage comprenant notamment la préparation de séquences d'acides nucléiques appropriées, leur ligation et leur introduction dans un vecteur d'expression adapté, par exemple un plasmide, et la transfection du plasmide dans une cellule hôte apte à permettre l'expression et l'obtention des protéines recombinantes chimères.

Selon un autre mode de réalisation, une entité et une protéine convenant à l'invention peuvent être associées l'une à l'autre au moyen d'une ou d'interaction(s) spécifique(s) de forte(s) affinité(s).

Un procédé de criblage selon l'invention peut mettre en oeuvre un transfert d'énergie de fluorescence (FRET), un transfert d'énergie de bioluminescence (BRET), ou la résonance plasmonique de surface (SPR), et plus particulièrement peut mettre en oeuvre un transfert d'énergie de fluorescence.

Selon un mode de réalisation, une entité convenant en particulier à l'invention peut être un anticorps ou une combinaison d'anticorps primaire et secondaire.

Un anticorps convenant à l'invention peut être obtenu par tout procédé connu de l'homme de l'art, notamment comme décrit dans « Antibodies : A Laboratory Manual », Cold Spring Harbor Laboratory Press, Cold Spring Harbor, N.Y. (1990).

A titre de type d'anticorps convenant à l'invention, on peut citer les anticorps monoclonaux, polyclonaux ou recombinants, ainsi que des fragments d'immunoglobuline susceptibles de lier un antigène et qui peuvent être produits par toute technique de génie génétique connue de l'homme de l'art ou par coupure enzymatique ou chimique d'anticorps intacts.

Plus particulièrement, un anticorps convenant à l'invention peut être choisi parmi un anticorps monoclonal, un anticorps polyclonal, un « diabody », un anticorps scFv, un anticorps F(ab')², un anticorps Fab', un anticorps chimérique, un anticorps humanisé et un anticorps recombinant.

Selon un mode de réalisation, un anticorps convenant à l'invention peut présenter une affinité pour une partie de la protéine contre laquelle il est dirigé ou pour un marqueur d'affinité spécifique porté par la protéine. En particulier, un anticorps convenant peut être un anticorps comme décrit dans les exemples détaillés ci-après.

Selon un mode de réalisation de la présente invention, une ou les protéine(s) de l'invention peuvent porter un marqueur d'affinité reconnu par un anticorps.

Un marqueur d'affinité convenant à l'invention peut notamment être choisi parmi les marqueurs d'affinité précédemment indiqués.

Selon un autre mode de réalisation, la première et la seconde protéines sont associées, respectivement, à une première et une seconde entités, lesdites entités comportant, respectivement, un groupe fluorescent A et un groupe fluorescent B, lesdits groupes A et B définissant un couple accepteur-donneur d'énergie de fluorescence convenant à la mise en oeuvre d'un transfert d'énergie par résonance de fluorescence.

A titre d'exemples de couples accepteur-donneur utilisables selon la présente invention, on peut citer, de façon non limitative, les couples BFP (Blue Fluorescent Protein)/GFP (Green Fluorescent Protein), CFP (Cyan Fluorescent Protein)/YFP (Yellow Fluorescent Protein), CFP/DsRed (Red Fluorescent Protein), GFP/DsRed et EuK (Cryptate d'Europium)/XL665. De préférence, un couple accepteur-donneur convenant à l'invention peut être EuK/XL665.

Selon une variante de réalisation, les première et seconde entités peuvent être des marqueurs fluorescents. De tels marqueurs fluorescents peuvent être choisis parmi des molécules fluorescentes ou des protéines fluorescentes habituellement mise en oeuvre dans le domaine. De manière préférée, les marqueurs fluorescents utilisés peuvent être choisis parmi des protéines fluorescentes fusionnées par biologie moléculaire, notamment comme indiqué précédemment, à une protéine de l'invention.

Selon une autre variante de réalisation, les première et seconde entités peuvent être des anticorps portant des marqueurs fluorescents et peuvent être aptes à se fixer, respectivement, sur un premier et un second marqueurs d'affinité portés, respectivement, par lesdites première et seconde protéines.

Selon un mode de réalisation, le signal S1 déterminé quantitativement ou qualitativement à l'étape d) du procédé selon l'invention peut être un signal fluorescent obtenu par irradiation à une longueur d'onde excitatrice du donneur d'énergie de fluorescence.

Le signal S2, déterminé quantitativement ou qualitativement à l'étape f) du procédé, peut être un signal fluorescent obtenu par irradiation à une longueur d'onde excitatrice du donneur d'énergie de fluorescence.

Les signaux de fluorescence S1 et S2 peuvent être mesurés à l'aide de tout dispositif adapté à l'invention et connu de l'homme de l'art. A titre d'exemples de dispositifs convenant à l'invention, on peut citer les lecteurs de microplaques, les microscopes à fluorescence, les spectrofluoromètres, ou les scanners à fluorescence.

Selon un mode de réalisation préféré, un procédé de l'invention peut être effectué en haut-débit, dans des microplaques, et la mesure des signaux de fluorescence peut être effectuée au moyen d'un lecteur de microplaques, par exemple un lecteur PHERAstar (BMG).

Un signal S2 significativement plus intense que le signal S1, en présence d'un composé à cribler peut être indicatif d'un composé favorisant ou agoniste de l'interaction SASPase-FLG2.

En revanche, un signal S2 significativement plus faible que le signal S1 en présence d'un composé à cribler peut être indicatif d'un composé déstabilisant ou antagoniste de l'interaction SASPase-FLG2.

Un composé criblé par un procédé ou une utilisation selon l'invention peut être un agoniste ou un antagoniste de l'interaction entre les première et seconde protéines.

Selon un mode de réalisation préféré de l'invention, un composé criblé peut être apte à traiter et/ou prévenir un désordre esthétique ou un désordre pathologique de la peau et/ou de ses annexes lié à un défaut de la différentiation et/ou de la prolifération des cellules de l'épiderme.

Selon un mode de réalisation, un procédé de criblage de l'invention peut comprendre en outre au moins une étape consistant à évaluer la propriété d'un composé criblé à moduler la différenciation et/ou la prolifération des cellules d'un épiderme.

Une telle évaluation peut être effectuée *in vitro* ou *ex vivo,* notamment sur des cellules d'épiderme en culture. Les cellules peuvent être des cellules en lignées, c'est-à-dire transformées par un virus ou d'origine tumorale pour les immortaliser. Alternativement, les cellules peuvent être des cellules de culture primaire, c'est-à-dire issues d'un tissu prélevé chez un organisme vivant, par exemple un épiderme d'un être humain. De préférence, les cellules en culture peuvent être des kératinocytes, voire associés à des fibroblastes, notamment stratifiés. Les cultures de cellules peuvent être effectuées selon toute méthode connue de l'homme de l'art.

Egalement, une évaluation *in vitro* peut être effectuée sur un modèle d'épiderme reconstruit. De tels modèles d'épiderme peuvent être disponibles commercialement, par exemple comme le modèle Episkin®.

L'évaluation *in vitro* ou *ex vivo* d'un composé criblé selon l'invention peut être effectuée par tout protocole connu de l'homme de l'art visant à comparer l'effet donné d'un composé à tester à une valeur contrôle ou standard.

L'effet d'un composé criblé selon l'invention, *in vitro* ou *ex vivo,* peut être déterminé par la mesure de marqueurs biologiques connus comme étant spécifiquement associés à la différentiation et/ou la prolifération des cellules d'un épiderme.

De tels marqueurs peuvent être choisis, par exemple, parmi la mesure de l'épaisseur du *stratum corneum*, de sa fonction barrière, la mesure de l'expression et/ou de l'activation de la transglutaminase I, de la Filaggrine, de la Caspase 14, de la SASPase, de la Cornéodésmosine, de la Desmogléine 1 ou de la protéine Ki67.

Ces marqueurs peuvent être déterminés, par exemple, par qPCR, par Western Blost ou immunofluorescence selon tout protocole connu de l'homme de l'art.

### Indications

Un composé de l'invention peut avantageusement être utilisé en vue de traiter et/ou prévenir dans la peau et/ou ses annexes un déséquilibre de la différentiation et/ou de la prolifération des cellules d'un épiderme.

L'invention vise l'ensemble de la peau du corps, incluant le cuir chevelu et les muqueuses, et de manière préférée, la peau du visage, du décolleté, du cou, des bras et avant-bras et les lèvres, voire de manière plus préférée encore, la peau du visage, du décolleté et du cou et les lèvres.

Selon un autre mode de réalisation, la présente invention concerne les cheveux, les poils, les cils et les ongles. De manière préférée, l'invention vise plus particulièrement les cheveux, les cils et les poils.

Selon un mode de réalisation, un défaut esthétique considéré par l'invention peut être choisi parmi des signes cutanés du vieillissement, un désordre de la fonction barrière de l'épiderme, des signes de sécheresse cutanée, un désordre de la desquamation de la peau, un défaut de cicatrisation, et/ou de ré-épithélialisation.

Selon un autre mode de réalisation, un défaut esthétique considéré par l'invention peut être choisi parmi une hypersudation et/ou un problème d'odeur corporelle.

Selon un autre mode de réalisation, un composé de l'invention peut être utilisé en cosmétique à titre d'agent actif pour favoriser la ré-épithélialisation après un traitement de gommage cutané ou exfoliant.

Un composé modulateur de l'invention permet donc avantageusement de restaurer plus rapidement les propriétés barrières d'un épiderme après un traitement cosmétique susceptible d'être agressif, et d'améliorer sa protection.

Selon un autre mode de réalisation, un composé de l'invention peut être utilisé en cosmétique à titre d'agent actif pour prévenir et/ou traiter la chute des cheveux et/ou favoriser la repousse des cheveux.

Selon un autre mode de réalisation, un composé de l'invention peut être utilisé en cosmétique à titre d'agent actif pour prévenir la repousse des poils.

Selon un autre mode de réalisation, un désordre pathologique peut être choisi parmi le cancer, le psoriasis, le vitiligo, la rosacée, et la dermatite atopique.

Les signes cutanés du vieillissement plus particulièrement considérés par l'invention peuvent être toutes modifications de l'aspect extérieur de la peau dues au vieillissement qu'il soit chronologique et/ou photo-induit, comme par exemple les rides et ridules, la peau flétrie, la peau relâchée, le manque d'élasticité et/ou de tonus de la peau, l'amincissement du derme et/ou la dégradation des fibres de collagène, ce qui entraîne l'apparence de peau molle et ridée. Sont également considérées toutes les modifications internes de la peau qui ne se traduisent pas systématiquement par un aspect extérieur modifié, comme par exemple toutes les dégradations internes de la peau, particulièrement des fibres d'élastine, ou fibres élastiques, consécutives à une exposition aux rayonnements ultraviolets.

En particulier, les signes cutanés du vieillissement visés par l'invention concernent un amincissement d'un épiderme et/ou une perte de fermeté, d'élasticité, de densité et/ou de tonicité d'un épiderme et/ou la formation de rides et ridules.

Un désordre de la fonction barrière d'un épiderme considéré par l'invention peut en particulier résulter d'agressions externes de type agents irritants, tels que par exemple les détergents, acides, bases, oxydants, réducteurs, solvants concentrés, gaz ou fumées toxiques, sollicitations mécaniques, telles que par exemple les frottements, chocs, abrasions, en particulier à l'issu d'un procédé d'exfoliation, arrachement de la surface de l'épiderme, projection de poussières, de particules, rasage ou épilation, les déséquilibres thermiques ou climatiques, tels que par exemple le froid, sécheresse, radiations, xénobiotiques, notamment les micro-organismes indésirables, allergènes, ou d'agressions internes de type stress psychologique.

Un composé modulateur de l'invention permet donc d'améliorer les propriétés barrières et la protection d'un épiderme.

Un désordre de la fonction barrière d'un épiderme peut notamment se traduire par un inconfort cutané, des phénomènes sensoriels et notamment des phénomènes désagréables, notamment des picotements, des tiraillements, des échauffements, et des démangeaisons.

Sous une forme pathologique, un désordre de la fonction barrière peut se manifester sous la forme d'une dermatite atopique.

Les signes cutanés de sécheresse plus particulièrement considérés par l'invention peuvent être toutes les modifications de l'aspect extérieur d'un épiderme, et notamment d'une peau, dues à sa déshydratation. Une peau sèche apparaît rugueuse au toucher, écailleuse ou couverte de squames, et se manifeste pour l'essentiel par une sensation de tiraillement et/ou de tension, liée à une souplesse et une élasticité diminuées.

Une peau sèche peut être pathologique ou non pathologique. Une peau sèche pathologique peut être représentée par la dermatite atopique et les ichthyoses.

Une peau sèche non pathologique peut être représentée par la peau sénile, notamment caractérisée par une diminution générale du métabolisme cutané avec l'âge, la peau fragile, pouvant se caractériser par une peau très sensible aux facteurs extérieurs et souvent accompagnée d'érythème et de rosacée, et la xérose vulgaire, d'origine génétique probable et se manifestant en priorité sur le visage, les membres et le dos des mains.

Un désordre de la desquamation plus particulièrement considéré dans l'invention peut être un désordre de la desquamation de la peau ou du cuir chevelu.

Un désordre de la desquamation du cuir chevelu peut apparaître sous la forme d'un état pelliculaire, notamment sec.

Les pellicules sèches sont des squames de petites tailles, se détachant facilement de l'épiderme qui, à l'examen clinique, n'apparaît pas inflammatoire.

Les défauts de cicatrisation et de ré-épithélialisation considérés par l'invention peuvent notamment se manifester sous la forme d'altérations de l'épiderme ou de défauts cicatriciels de l'épiderme résultants d'interventions chirurgicales ou consécutifs à un désordre esthétique ou pathologique de l'épiderme, telle que des cicatrices d'acné ou des signes de vergetures. Les défauts de ré-épithélialisation considérés par l'invention peuvent également survenir suite à une exfoliation chimique ou mécanique de la peau ou dans le cas des peaux âgées. Un composé modulateur peut être utilisé avant ou après l'exfoliation pour favoriser le rétablissement des fonctions barrières de l'épiderme.

Les odeurs corporelles considérées par l'invention peuvent notamment être toute odeur indésirable susceptible d'être perçue comme gênante, voire invalidante, par l'individu la manifestant et/ou son entourage.

Il s'agit plus particulièrement d'odeur(s) constatée(s) au niveau des zones du corps de forte sudation et plus particulièrement les zones axillaires, notamment les aisselles, ou bien encore les pieds, les auréoles mammaires et les régions péri-anales, où se trouve la majeure partie des glandes sudoripares, en particulier apocrines.

Les odeurs corporelles sont notamment celles relevant d'une sudation, voire hypersudation, et/ou de la dégradation bactérienne de la sueur et plus particulièrement de la sueur apocrine.

### Compositions

Un composé de l'invention est avantageusement formulé dans une composition pouvant se présenter sous toutes formes galéniques normalement disponibles pour l'indication et le mode d'administration retenu.

Une composition selon l'invention comprend un milieu physiologiquement ou pharmaceutiquement acceptable.

Une composition selon l'invention peut être administrée par voie orale, parentérale ou topique.

De manière préférée, une composition de l'invention peut être administrée par voie topique.

Selon un mode de réalisation, une composition par voie topique selon l'invention peut avantageusement être formulée sous toute forme galénique convenant au soin de la peau et des muqueuses et peuvent se présenter sous forme d'onguents, de crèmes, de laits, de pommades, de poudres, de tampons imbibés, de solutions, de gels, de sprays, de lotions, ou de suspensions. Elles peuvent également se présenter sous forme de microsphères ou nanosphères ou de vésicules lipidiques ou polymériques ou de patchs polymériques et d'hydrogels permettant une libération contrôlée. Ces compositions par voie topique peuvent se présenter soit sous forme anhydre, soit sous forme aqueuse selon l'indication dermocosmétique.

Une composition destinée à une administration par voie topique peut être une solution aqueuse, hydroalcoolique ou huileuse, une solution ou une dispersion du type lotion ou sérum, une émulsion de consistance liquide ou semi-liquide du type lait, obtenue par dispersion d'une phase grasse dans une phase aqueuse (H/E) ou inversement (E/H), une suspension ou une émulsion, de consistance molle, semi-solide ou solide, du type crème ou du type gel aqueux ou anhydre, une émulsion multiple (E/H/E ou H/E/H), une microémulsion, une nano-émulsion, une préparation de microcapsules, une préparation de microparticules, ou une dispersion vésiculaire de type ionique et/ou non ionique, ou une dispersion cire/phase aqueuse.

Selon un mode préféré de réalisation, une composition par voie topique peut se présenter sous la forme d'une solution, d'une crème, d'un gel, d'une émulsion, d'une mousse ou d'une composition pour aérosol contenant un agent propulseur.

Selon un mode préféré de réalisation, une composition par voie topique peut également se présenter sous la forme d'un système transdermique permettant une libération active ou passive du/des actif(s) par transdermie, par exemple de type patch ou gel patch (hydrogel).

Ces compositions sont préparées selon les méthodes usuelles.

Une composition selon l'invention peut constituer une composition de traitement ou de soin de la peau ou du cuir chevelu, ou une composition de protection solaire ou de bronzage artificiel, ou encore un produit nettoyant ou démaquillant de la peau, un produit déodorant ou encore un composé parfumant, une crème de nettoyage, de protection, de traitement ou de soin, une lotion, un gel ou une mousse pour le soin de la peau, une lotion de nettoyage ou de désinfection, une composition pour le bain ou une composition déodorante. Elle peut également se présenter sous forme de shampooing traitant ou non, colorant ou non, ou d'après-shampooing. Une composition selon l'invention peut également consister en une préparation solide constituant un savon ou un pain de nettoyage.

Une telle composition peut être alors non colorée ou faiblement colorée, et peut contenir éventuellement des actifs cosmétiques ou dermatologiques additionnels, notamment comme indiqué ci-après. Elle peut alors être utilisée comme base de soin pour la peau ou les lèvres, par exemple sous forme d'un baume à lèvres, protégeant les lèvres du froid et/ou du soleil et/ou du vent, comme une crème de soin de jour ou de nuit pour la peau du visage et/ou du corps.

Une composition selon l'invention peut également constituer une composition cosmétique colorée et notamment une composition de maquillage de la peau et/ou des muqueuses, en particulier une telle composition peut être un fond de teint, un blush, un fard à joues ou à paupières, un composé anti-cernes en stick, un rouge à lèvres ou un brillant à lèvres, présentant éventuellement des propriétés de soin ou de traitement. De préférence, il pourra s'agir d'une composition de maquillage colorée (beige ou verte) destinée à corriger la couleur du teint.

Une composition selon l'invention peut également constituer une composition de maquillage ou de soin des ongles ou des cils.

Lorsqu'une composition de l'invention est une émulsion, la proportion de la phase grasse peut aller de 5 à 80 % en poids, et de préférence de 10 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique et/ou dermatologique. L'émulsionnant et le co-émulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

Dans le cas d'une composition conforme à l'invention pour une administration par voie orale, on privilégie l'utilisation d'un support ingérable.

Le support ingérable peut être de nature diverse selon le type de composition considérée.

Conviennent ainsi, notamment, comme supports alimentaires des comprimés, des gélules ou des tablettes, des suspensions, des suppléments oraux sous forme sèche et des suppléments oraux sous forme liquide.

Conviennent également comme supports alimentaires, le lait, le yaourt, le fromage, les laits fermentés, les produits fermentés à base de lait, des glaces, des produits à base de céréales ou des produits à base de céréales fermentées, des poudres à base de lait, des formules pour enfants et nourrissons, des produits alimentaires de type confiserie, le chocolat, les céréales, ou des aliments pour animaux en particulier domestiques.

Par composition pour voie orale, on entend par exemple des compositions nutritionnelles, nutraceutiques, cosméceutiques, ou pharmaceutiques, comprenant au moins un composé selon l'invention.

La formulation des compositions pour voie orale selon l'invention peut être réalisée par tout procédé usuel connu de l'homme de l'art pour produire des solutions buvables, des dragées, des gélules, des gels, des émulsions, des comprimés à avaler ou à croquer, des capsules, notamment des capsules molles ou dures, des granulés à dissoudre, des sirops, des aliments solides ou liquides et des hydrogels permettant une libération contrôlée, des barres alimentaires, des poudres, compactées ou non, des suspensions ou solutions liquides, des confiseries, du lait fermenté, des fromages fermentés, des chewing-gums, des pâtes dentifrices ou des solutions spray.

Les compositions par voie orale peuvent se présenter soit sous forme anhydre, soit sous forme aqueuse selon l'indication dermocosmétique.

Un composé de l'invention peut être par ailleurs formulé avec les excipients et composants usuels pour de telles compositions orales ou compléments alimentaires, à savoir notamment des composants gras et/ou aqueux, des agents humectants, des agents épaississants, des agents conservateurs, des agents de texture, de saveur et/ou d'enrobage, et/ou des agents antioxydants.

Les agents de formulation et excipients pour composition orale, et notamment pour compléments alimentaires, sont connus dans ce domaine et ne font pas ici l'objet d'une description détaillée.

En particulier, une composition selon l'invention peut être une composition alimentaire pour la consommation humaine. Il peut s'agir en particulier d'aliments complets nutritionnels, de boissons, d'eaux minérales, de soupes, de suppléments diététiques et d'aliments de substitution, de barres nutritionnelles, de confiseries, de produits à base de lait fermenté ou non, de yaourts, de poudres à base de lait, de produits de nutrition entérale, de compositions pour enfants et/ou nourrissons, de produits à base de céréales fermentées ou non, de glaces, de chocolat, de café, de produits « culinaires » tels que de la mayonnaise, la purée de tomate, ou des assaisonnements pour salades.

La composition selon l'invention peut également être destinée aux animaux, notamment aux animaux domestiques, tels que les chats et les chiens, et être formulée sous forme d'aliments ou de compléments alimentaires pour animaux.

Selon un autre mode de réalisation, un composé convenant à l'invention peut être mis en oeuvre par voie parentérale, en particulier par voie sous-cutanée ou intradermique.

Dans une telle mise en oeuvre, un actif convenant à l'invention peut être conditionné sous la forme d'une solution isotonique stérile aqueuse ou non aqueuse, sous forme de dispersion, de suspension ou d'émulsion préparée le cas échéant juste avant l'administration, à partir d'une poudre stérile, par exemple lyophilisée, qui est alors reconstituée sous la forme d'une solution stérile injectable ou d'une dispersion au moment de son utilisation.

Par « stérile », on entend qualifier une formulation apte à garantir l'innocuité requise pour une administration intraépidermique et/ou intradermique et/ou sous-cutanée.

Une composition convenant à l'invention peut comprendre tout excipient habituellement utilisé dans le domaine des solutions stériles injectables.

L'administration d'une composition de l'invention par voie parentérale peut être effectuée par toute technique et/ou dispositif d'injection convenant à une injection intraépidermique et/ou intradermique et/ou sous-cutanée. Ainsi, une telle administration peut être effectuée par mésothérapie.

Pour la voie parentérale, on pourra privilégier l'administration par patch à visée systémique.

Une composition selon l'invention peut en outre comprendre au moins un ingrédient cosmétiquement ou dermatologiquement acceptable. Les quantités de ces différents ingrédients sont celles classiquement utilisées dans le domaine considéré, et sont notamment déterminées pour ne pas affecter les propriétés recherchées pour un composé de l'invention ou pour une composition de l'invention.

Un actif cosmétique ou dermatologique convenant à l'invention peut être choisi parmi des hydratants, des vitamines, des acides gras essentiels, des sphingolipides, des filtres UV, des composés antioxydants, des agents anti-acnés, des agents anti-inflammatoires, des agents bronzant (en l'absence de rayonnement UV), des agents dépigmentant, des agents matifiant, des protéines ou des hydrolysats de protéines, des acides aminés, des polyols, l'urée, l'allantoïne, des sucres et des dérivés de sucre, des vitamines hydrosolubles, des extraits végétaux et des hydroxyacides, le rétinol et ses dérivés, le tocophérol et ses dérivés, des acides gras essentiels, des céramides, des huiles essentielles, l'acide salicylique et ses dérivés, la vitamine D3 et ses dérivés, les rétinoïdes et leurs dérivés, les agonistes PPAR-gamma, les agents antichute ou repousse pour les cheveux comme les ouvreurs de canaux potassiques, et les anti-androgènes, et leurs mélanges.

Une composition de l'invention peut comprendre tout agent de formulation habituellement utilisée dans le domaine.

Comme gélifiants hydrophiles convenant à l'invention, on peut citer les polymères carboxyliques tel que le carbomer, les copolymères acryliques tels que les copolymères d'acrylates/alkylacrylates, les polyacrylamides et notamment le mélange de polyacrylamide, C₁₃₋₁₄ Isoparaffine et Laureth-7 vendu sous le nom de SEPIGEL 305® par la société SEPPIC, les polysaccharides comme les dérivés cellulosiques tels que les hydroxyalkylcelluloses et en particulier les hydroxypropylcelluloses et hydroxyéthylcelluloses, les gommes naturelles telles que les guar, caroube et xanthane et les argiles.

Comme gélifiants lipophiles convenant à l'invention, on peut citer les argiles modifiées comme les bentones, les sels métalliques d'acides gras comme les stéarates d'aluminium et la silice hydrophobe, ou encore l'éthylcellulose et le polyéthylène.

Comme agents tensioactifs utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination SINNOWAX AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20 OE).

Comme charges convenant à l'invention on peut citer le talc, le mica, la silice, le kaolin, le carbonate de calcium précipité, le carbonate et l'hydrogéno-carbonate de magnésium, l'hydroxyapatite, le nitrure de bore, les poudres de polyamide (NYLON® ORGASOL de chez ATOCHEM), de poly-b-alanine et polyéthylène, les poudres de polytétrafluoroéthylène (TEFLON®), la lauroyl-lysine, l'amidon, les poudres de polymères de tétrafluoroéthylène, les microsphères creuses de polymères telles l'EXPANCEL (NOBEL INDUSTRIE), les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (CHEMDAL CORPORATION), les microbilles de résine de silicone (TOSPEARL® de TOSHIBA, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI, et leurs mélanges.

Comme absorbeurs d'odeurs convenant à l'invention, on peut citer les zéolites et les cyclodextrines.

Une matière colorante convenant à l'invention est un composé susceptible de produire un effet optique coloré lorsqu'il est formulé en quantité suffisante dans un milieu cosmétique approprié. A titre de matière colorante utilisable, on peut citer les pigments, les nacres, les paillettes, les colorants liposolubles, les colorants hydrosolubles, et leurs mélanges.

Les pigments peuvent être choisis parmi les pigments minéraux, les pigments organiques, et les pigments composites (c'est-à-dire des pigments à base de matériaux minéraux et/ou organiques). Les pigments peuvent être choisis parmi les pigments monochromes, les laques, les nacres, les pigments à effet optiques, comme les pigments réfléchissants et les pigments goniochromatiques.

Un polymère filmogène convenant à l'invention peut être choisi parmi les polymères radicalaires, les polycondensats, les polymères d'origine naturelle, et leurs mélanges. On entend par « polymères » des composés comportant au moins deux motifs, de préférence au moins 3 motifs et plus spécialement au moins 10 motifs de répétition.

Les polymères radicalaires peuvent être, par exemple, choisis parmi les polymères acryliques et/ou les polymères vinyliques. Selon l'invention, les polycondensats peuvent être choisis parmi les polyuréthannes, les polyesters, les polyesters amides, les polyamides, les polyesters à chaîne grasse, les résines époxyesters et leurs mélanges. Comme polymère d'origine naturelle, on peut citer la shellac et la gomme de sandaraque.

Par « polymère semi-cristallin », on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Un polymère semi-cristallin peut être choisi parmi les copolymères séquencés comportant au moins une séquence cristallisable et au moins une séquence amorphe, les homopolymères, les copolymères portant au moins une chaîne latérale cristallisable par motif de répétition, et leurs mélanges. De tels polymères sont décrits par exemple dans le document EP 1 396 259. A titre d'exemple particulier de polymère semi-cristallin structurant utilisable selon l'invention, on peut citer les produits INTELIMER® de la société LANDEC décrits dans la brochure « INTELIMER® POLYMERS », LANDEC IP22 (Rev. 4-97). Ces polymères sont sous forme solide à température ambiante (25 °C). Ils sont porteurs de chaînes latérales cristallisables.

Comme matières grasses utilisables dans l'invention, on peut citer les huiles volatiles et les huiles non volatiles, comme par exemple les huiles minérales tel que le polyisobutène hydrogéné et l'huile de vaseline, les huiles végétales comme par exemple une fraction liquide du beurre de karité, l'huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés, les huiles siliconées et les huiles fluorées comme par exemple les perfluoropolyéthers.

Un corps gras pâteux ou solide pouvant être présent dans une composition de l'invention peut être, par exemple un acide gras choisi parmi les acides gras comportant de 8 à 30 atomes de carbone, comme l'acide stéarique, l'acide laurique, l'acide palmitique et l'acide oléique ; une cire choisi parmi les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; une gomme choisie parmi les gommes de silicone (diméthiconol), un composé pâteux, comme les composés siliconés polymériques ou non, les esters d'un glycérol oligomère, le propionate d'arachidyle, les triglycérides d'acides gras et leurs dérivés, et leurs mélanges.

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de VITTEL, les eaux du bassin de VICHY et l'eau de la ROCHE POSAY.

### Article de conditionnement

Un article de conditionnement de l'invention sera naturellement choisi par l'homme de l'art en fonction de la forme galénique de la composition à conditionner.

Ainsi, pour des compositions liquides, pourront être utilisés des containers constitués d'une enveloppe rigide comprenant des moyens de distribution de la composition. Ces moyens de distribution peuvent être un simple orifice dont l'obturation est assurée par un opercule amovible, ou un bouton poussoir associé à une pompe permettant d'expulser une partie de la composition contenue dans le container. Egalement, les compositions de l'invention sous forme liquide peuvent être conditionnées dans des containers de type flacons aérosols.

Une composition de l'invention sous forme semi-liquide ou pâteuse peut être avantageusement conditionnée dans un pot, un tube de crème, ou dans un container à parois souples ou déformables et dotée d'un orifice obturable par un opercule amovible, la composition étant expulsée au travers de l'orifice par pression sur les parois, ou un flacon doté d'un bouton poussoir et d'une pompe comme indiqué précédemment.

Selon un mode de réalisation particulier, un article de conditionnement apte à accueillir une composition selon l'invention peut être réalisé en verre, métal, alliage, papiers couchés tels que les papiers enduits de cires comme par exemple les papiers enduits de cire d'abeille, qui présentent notamment des propriétés comme conservateur naturel.

Alternativement, une composition selon l'invention peut être stockée sous vide, dans un compartiment imperméable à l'air, fermé hermétiquement, à l'image par exemple d'une brique sous vide, communément utilisée dans le domaine alimentaire.

L'article de conditionnement peut être réalisé au moins en partie en matières plastiques ou autres matériaux polymériques adéquat.

Selon un mode de réalisation particulier, une composition selon l'invention peut être conditionnée « sous atmosphère protectrice ». Un conditionnement « sous atmosphère protectrice » consiste à modifier la composition de l'atmosphère interne d'un emballage dans le but d'améliorer la durée de vie de son contenu. Cette technique consiste à réduire le taux d'oxygène afin de ralentir la croissance des formes de vie aérobie et les réactions d'oxydation. L'oxygène enlevé peut être remplacé par d'autres gaz.

Selon un mode de réalisation particulier, l'article de conditionnement peut également être réalisé à l'aide de matériaux permettant d'isoler la composition de toutes sources lumineuses.

L'article de conditionnement peut également être au moins pour partie réalisé en matériaux d'isolation thermique. A titre d'exemples de ce type de matériaux, on peut citer les tissus, les tissus en fibres de verre enduits de silicone, les textiles à base de fibres céramiques, les fibres de cellulose, le polystyrène, la styromousse, et les films d'emballage. On peut également utiliser comme matériau d'isolation thermique, un gel ou liquide froid.

Selon un mode de réalisation particulier, l'article de conditionnement est à usage unique et ouvert juste avant son utilisation par le consommateur.

Selon un mode de réalisation, une composition de l'invention formulée à l'état solide peut être, par exemple, conditionnée dans un pot, un tube pour stick, par exemple un tube pour rouge à lèvres.

### Procédé de traitement cosmétique

Comme indiqué précédemment, l'invention concerne un procédé cosmétique dédié aux individus présentant des défauts esthétiques de la peau et/ou de ses annexes lié à un déséquilibre de la différentiation et/ou de la prolifération des cellules de l'épiderme.

Un individu concerné par un procédé de traitement cosmétique de l'invention est naturellement un individu présentant, ou susceptible de présenter, au moins une des indications de soins cosmétiques précédemment définies.

Un procédé de l'invention permet de traiter un défaut esthétique de la peau et/ou de ses annexes tel que défini précédemment.

Un procédé selon l'invention peut comprendre, en outre, une étape consistant à observer une réduction voire une disparition du défaut esthétique de la peau et/ou de ses annexes, ou encore une amélioration de l'aspect esthétique de la peau et/ou de ses annexes.

De préférence, un procédé selon l'invention peut être mis en oeuvre par voie topique, orale ou parentérale.

Encore plus préférentiellement, un procédé selon l'invention est mis en oeuvre par voie topique ou orale.

Un procédé selon l'invention peut être mis en oeuvre, par voie topique, notamment par application sur la peau et/ou ses annexes d'au moins une couche d'une composition cosmétique ou dermatologique comprenant à titre d'actif au moins un composé de l'invention, et plus particulièrement d'une composition cosmétique telle que définie précédemment.

Selon un mode de réalisation, un procédé de l'invention peut comprendre une étape d'application d'au moins une couche d'une composition cosmétique ou dermatologique, comprenant à titre d'actif au moins un composé de l'invention, sur la peau du corps, notamment la peau du visage ou du décolleté, sur le cuir chevelu, sur les lèvres, sur les cheveux, les cils ou les ongles.

De manière préférée, un procédé de l'invention peut comprendre l'application topique sur la peau ou les lèvres, notamment la peau du visage et du décolleté, d'au moins une couche d'une composition de l'invention.

Avantageusement, un procédé de l'invention par voie topique peut comprendre l'application d'une composition conforme à l'invention, par exemple sous forme de masque, notamment sur la peau du visage.

Une telle administration peut être effectuée selon les techniques d'utilisation habituelles de ces compositions. Par exemple, elle peut consister en l'application de crèmes, de gels, de sérums, de lotions sur la peau ou les muqueuses.

Un procédé cosmétique topique selon l'invention peut être mis en oeuvre de façon journalière par exemple, à raison par exemple d'une administration unique par jour ou d'une administration deux fois par jour, par exemple une fois le matin et une fois le soir.

Un procédé cosmétique topique selon l'invention peut être mis en oeuvre sur une période de temps variant d'une semaine à plusieurs semaines, voire plusieurs mois, cette période pouvant par ailleurs être répétée après des périodes de non traitement, pendant plusieurs mois voire plusieurs années.

A titre d'exemple, l'administration par voie topique d'un composé selon l'invention peut être répétée, par exemple, 2 à 3 fois par jour, ou plus, et généralement sur une durée prolongée d'au moins 4 semaines, voire 4 à 15 semaines, avec le cas échéant une ou plusieurs périodes d'interruption.

Un procédé de l'invention peut avantageusement comprendre l'application d'une composition de l'invention en association, de manière simultanée, successive ou séparée dans le temps, avec l'administration d'une composition cosmétique ou dermatologique additionnelle, distincte de la composition de l'invention, et destinée au soin ou au maquillage de la peau et/ou de ses annexes.

Toute composition cosmétique ou dermatologique additionnelle peut convenir à l'invention, sous réserve naturellement que son association avec une composition de l'invention n'affecte pas les propriétés recherchées pour cette dernière. L'homme de l'art sait apprécier sur la base de ses connaissances les compositions cosmétiques ou dermatologiques additionnelles susceptibles de convenir.

Selon un mode de réalisation, cette composition additionnelle peut être administrée par voie topique sur la peau et/ou ses annexes.

Selon un autre mode de réalisation, un procédé de l'invention peut comprendre l'application sur la peau et/ou ses annexes d'une composition de l'invention en association avec une application successive, par voie topique sur ladite peau et/ou lesdites annexes, d'une composition additionnelle cosmétique ou dermatologique.

La composition de l'invention et la composition additionnelle peuvent être appliquées l'une sur l'autre selon une gestuelle cosmétique dite de « double geste ».

Selon un mode de réalisation, un procédé selon l'invention peut comprendre l'application sur la peau et/ou ses annexes, à titre de première couche ou « couche de base » ou « base coat », d'une composition de l'invention et, à titre de seconde couche ou « couche supérieure » ou « top coat », d'une couche d'une composition additionnelle telle que définie précédemment.

Selon une variante de réalisation, la première couche peut comprendre la composition additionnelle, et la seconde couche peut comprendre une composition de l'invention.

Un procédé cosmétique de l'invention peut être mis en oeuvre par voie orale, notamment, par administration d'au moins une composition alimentaire à visé cosmétique comprenant à titre d'actif au moins un composé de l'invention, et en particulier une composition alimentaire à visé cosmétique telle que définie ci-dessus.

Une composition selon l'invention peut être administrée en une dose unique par jour ou en doses fractionnées sur la journée, par exemple 2 à 3 fois par jour.

Un procédé cosmétique orale peut être mis en oeuvre sur une période de temps variant d'une semaine à plusieurs semaines, voire plusieurs mois, cette période pouvant par ailleurs être répétée après des périodes de non traitement, pendant plusieurs mois voire plusieurs années.

A titre d'exemple, l'administration par voie orale à titre d'actif d'un composé selon l'invention peut être effectuée à raison de, par exemple, 3 fois par jour, plus généralement sur une durée prolongée d'au moins 4 semaines, voire 4 à 15 semaines, comprenant ou non une ou plusieurs périodes d'interruption ou étant répétée après une période d'interruption.

Un procédé cosmétique selon l'invention peut également être mis en oeuvre par voie parentérale. La mise en oeuvre par voie parentérale d'un procédé cosmétique de l'invention exclut de fait toute intervention chirurgicale et ne vise qu'à exercer un traitement de surface de la peau à visée esthétique.

Une administration parentérale peut être effectuée, par exemple, par toute technique d'injection ou dispositif convenant à une injection intraépidermique et/ou intradermique et/ou sous-cutanée. Une telle administration peut être réalisée par mésothérapie.

Pour la voie parentérale, on pourra alternativement privilégier l'administration par patch à visée systémique.

A titre d'exemple, l'administration parentérale d'un composé de l'invention peut être effectuée, par exemple, au moins 1 fois par mois, voire au moins 1 fois par semaine, et plus généralement au moins 1 fois par jour, sur une durée plus ou moins prolongée pouvant être, par exemple, d'au moins 4 semaines, voire 4 à 15 semaines, comprenant ou non une ou plusieurs périodes d'interruption, ou étant répétée après une période d'interruption.

Selon un autre aspect, le présent brevet décrit l'utilisation d'une quantité efficace d'au moins un composé de l'invention pour la préparation et/ou l'amélioration d'un modèle cellulaire pluristratifié, notamment de type épidermique ou muqueux, et en particulier un modèle de peau reconstruite.

Avantageusement, un composé de l'invention est ajouté au milieu de culture dans lequel le modèle cellulaire pluristratifié est cultivé. Le moment et la durée d'incubation du modèle cellulaire en présence d'un composé de l'invention sont naturellement adaptés par l'homme de l'art en fonction du modèle cellulaire à obtenir et du composé utilisé.

Au sens de la présente description, on entend désigner par « modèle de peau reconstruite », un modèle dans lequel sont associés différents types cellulaires, tels que notamment les constituants naturels de la peau, à l'image par exemple des kératinocytes, des fibroblastes, des cellules de Langerhans et des mélanocytes. Les cellules de type fibroblastes peuvent être irradiées ou non.

De tels modèles et leur préparation sont connus de l'homme de l'art.

Dans la description et dans les exemples suivants, sauf indication contraire, les pourcentages sont des pourcentages en poids et les plages de valeurs libellées sous la forme « entre ... et ... » incluent les bornes inférieure et supérieure précisées.

Au sens de la présente invention, « un » doit se comprendre, sauf indication contraire, au sens de « au moins un ».

Les exemples figurant ci-après sont présentés à titre illustratif et non limitatif de l'invention.

### FIGURES

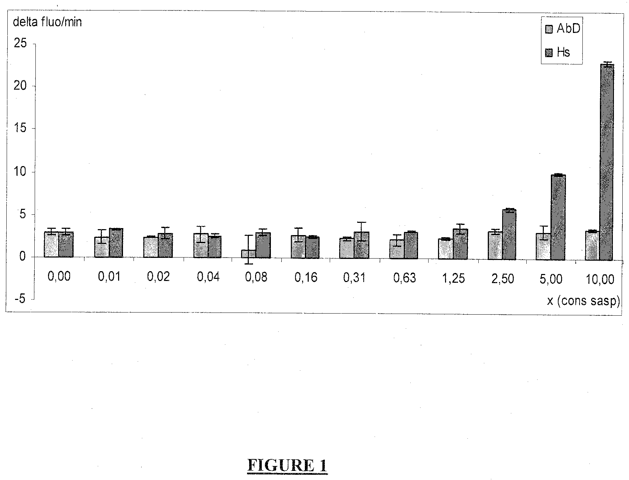

**Figure 1** **:** illustre l'évolution de l'hydrolyse enzymatique du substrat fluorescent Dabcyl-QIDRIMEK-Edans par une SASPase recombinante (28 kDa) en présence de concentrations croissantes de FLG2 recombinante active (complexe Hs = *Thioredoxine-His-S.Tag-FLG2₂₋₂₁₃ ;* histogramme noir) ou inactive (*complexe AbD* = *Thioredoxine-His-S.Tag-FLG2₈₁₋₂₁₂* ; histogramme gris) exprimées en multiple (0 ; 0,01 ; 0,02 ; 0,04 ; 0,06 ; 0,16 ; 0,3 ; 0,63 ; 1,25 ; 2,50 ; 5,00 et 10,00) de la concentration initiale de SASPase (0,025 mg/ml). La réaction enzymatique est réalisée dans un tampon acétate/acide acétique. La figure illustre la moyenne ± sem d'une expérience réalisée en duplicate.

### EXEMPLES

### Exemple 1

### Activation enzymatique de la SASPase par FLG2

### a- Matériels et méthodes

Deux formes de Filaggrine-2 (FLG2) recombinantes correspondant, respectivement, aux séquences d'acides aminés 2-213, forme active, et 81-212, forme inactive, de Q5D862 (réf. Uniprot/Swissprot) fusionnées, à leur extrémité N-terminale, à un marqueur d'affinité Thioredoxine-His-S.Tag (THX-His-S-tag) ont été préparées en plasmide parental pET32c (Novagen) ou pEB6 (HBGX) (FLG2₂₋₂₁₃-S-His-THX et FLG2₈₁₋₂₁₂-S-His-THX).

Ces protéines chimères recombinantes sont identifiées par la suite sous les dénominations FLG2-Hs = FLG2₂₋₂₁₃-S-His-THX (forme active) et FLG2-AbD = FLG2₈₁₋₂₁₂-S-His-THX (forme inactive).

Une SASPase recombinante de 28 kDa correspondant à la séquence d'acides aminés 85 à 343 de la séquence Q53RT3 (réf. Uniprot/Swissprot) a été préparée comme décrit par BERNARD et al. (J. Invest. Dermatol., 2005, 125 : 278-287) (SASPase₈₅₋₃₄₃).

La SASPase est mise en solution dans un tampon PBS et utilisée à une concentration finale de 0,025 mg/ml en présence d'une concentration finale d'un substrat colorimétrique Dabcyl-QIDRIMEK-Edans de 0,01 mM (Production Jerini Peptide Ref. D17 : JPT Peptide Technologies GmbH).

FLG2-Hs et FLG2-AbD sont mises en oeuvre aux concentrations correspondant à 0,01, 0,02, 0,04, 0,08, 0,16, 0,31, 0,63, 1,25, 2,5, 5 ou 10 fois la concentration finale de SASPase, i.e. 0,025 mg/ml.

La SASPase et FLG2-Hs, ou FLG2-AbD, sont incubées à 37° C dans un tampon acétate/acide acétique 0,1 M à pH 5,5 contenant 150 mM de NaCl, en présence de Dabcyl-QIDRIMEK-Edans.

L'hydrolyse du substrat colorimétrique par la SASPase activée conduit à la séparation du fluorochrome Edans et de son désactivateur (ou quencheur) Dabcyl, et à un accroissement de la fluorescence de Edans.

La mesure du signal fluorescent est effectuée à λₑₓ 340 nm/λₑₘ 490 nm. La fluorescence est lue toutes les 10 minutes pendant 1h30min, à 37 °C, avec un Spectramax M5e (Molecular Devices).

### b- Résultats

Les résultats obtenus montrent que l'activité enzymatique de la SASPase est significativement augmentée en présence de concentration croissante de FLG2-Hs active, alors qu'elle demeure pratiquement inchangée en présence de FLG2-AbD inactive (voir Figure 1).

Ces résultats montrent que FLG2 interagit avec la SASPase, et stimule son activité protéolytique. Une telle activation est obtenue à partir d'une concentration en FLG2 équivalente à celle de la SASPase.

### Exemple 2

### Criblage d'agents modulateurs de l'interaction SASPase-FLG2

L'interaction SASPase-FLG2, et la stimulation de l'activité protéolytique de la SASPase en découlant, ont été mises à profit dans un procédé de criblage de composés actifs aptes à moduler cette interaction.

Le procédé développé s'appuie sur l'interaction FLG2-SASPase et utilise la technique de fluorescence homogène en temps résolu (ou HTRF).

Une protéine SASPase recombinante (comprenant la séquence d'acides aminés 85-343 de Q53RT3) mutée dans son site actif (D212A de Q53RT3) et fusionnée avec le marqueur d'affinité GST (Glutathion S-Transférase de *Schistosoma japonicum*), à son extrémité C-terminale, a été préparée dans le vecteur pGEX-4-T3 (GenBank U13855) (SASPase_{85-343D212A}-GST). La mutation D212A a été introduite pour rendre la SASPase inactive et favoriser une interaction stable avec FLG2.

Une protéine FLG2 recombinante (comprenant la séquence d'acides aminés 2-213 de Q5D862) fusionnée au marqueur d'affinité Thioredoxine-His-S.Tag, à son extrémité N-terminale, a été préparée comme suit (FLG2₂₋₂₁₃-S-His-THX).

Les plasmides obtenus sont utilisés pour transformer des bactéries compétentes E. coli BL21DE3. Après transformation du plasmide correspondant à la protéine dans les bactéries, 4 ml de préculture ont été incubés une nuit à 37 °C sous agitation (250 rpm). La préculture a été ajoutée à 100 mL de milieu de culture (LB + Ampicilline) et l'ensemble est placé à 37 °C sous agitation jusqu'à obtenir une densité optique à 600 nm au moins égale à 0.8.

Les bactéries transformées sont alors induites en présence d'IPTG à 1 mM pendant une nuit à 16 °C sous agitation, puis centrifugées et lysées par ultrason. Les peptides chimères sont purifiés par affinité pour le tag HIS ou GST sur une résine appropriée, puis dialysées contre un tampon PBS 1x, pH 8. Les protéines chimères obtenues sont ensuite dosées par la méthode de Bradford.

Des anticorps reconnaissant, respectivement, les marqueurs d'affinité GST ou His-S.Tag, et porteurs, respectivement, d'un groupement donneur de fluorescence EuK (cryptate d'europium λₑₓ 337 nm/λₑₘ 620 nm) ou d'un groupement receveur de fluorescence XL1665 (λₑₓ 570-630 nm/λₑₘ 665 nm) sont utilisés pour suivre l'interaction peptide-SASPase.

De tels anticorps sont disponibles commercialement, par exemple auprès de la société Cisbio International sous les références 61GSTKLB (anticorps anti-GST K) ou 61HISKLB (anticorps anti-6HIS K).

L'interaction SASPase-FLG2 a pu être observée par transfert d'énergie de fluorescence (FRET) entre les marqueurs fluorescents Euk et XL1665. Le donneur de fluorescence Euk a été excité à λₑₓ 337 nm et l'émission de fluorescence de l'accepteur de fluorescence XL1665 a été mesurée à λₑₘ 665 nm.

L'émission de fluorescence Euk à λₑₘ 620 nm, ayant lieu indépendamment de l'interaction entre la SASPase et FLG2, a été utilisée pour normaliser le signal. Ainsi pour chaque point un ratio λₑₘ 665 / λₑₘ 620 est déterminé.

Le procédé de criblage a été effectué en plaque 384 puits au moyen d'un automate Janus de Perkin Elmer dans un tampon phosphate PBS à pH 7,4.

Les tests sont réalisés en quadruplicats. Le contrôle positif (100 % signal) est obtenu en présence des protéines recombinantes, DMSO et anticorps marqués avec les fluorophores. Le contrôle négatif (0 % signal ou background) est obtenu en présence de tampon de dilution des protéines (dénué de protéines), DMSO et anticorps marqués avec les fluorophores.

Le DMSO est utilisé à 2,5 % en concentration finale.

Les protéines recombinantes SASPase_{85-343D212A}-GST et FLG2₂₋₂₁₃-S-His-THX ont été utilisées aux concentrations finales respectives de 50 nM et 200 nM.

Les composés testés ont été mis en oeuvre aux concentrations finales de 5 10⁻⁹ M ; 1,5 10⁻⁸ M ; 4,6 10⁻⁸ M ; 1,4 10⁻⁷ M ; 4,1 10⁻⁷ M ; 1,2 10⁻⁶ M ; 1,1 10⁻⁵ M ; 3,3 10⁻⁵ M et 1 10⁻⁴ M.

Un tampon, ou une solution de dilution dénuée de composé, a été utilisée comme témoin.

La SASPase_{85-343D212A}-GST et les composés testés ont été pré-incubés à 4° C pendant 60 min, puis la FLG2-His-THX a été ajoutée, et l'ensemble a été laissé incuber à 4 °C sur la nuit.

Les anticorps spécifiques de chacune des protéines chimères ont ensuite été ajoutés, à la dilution finale de 1/400 dans un tampon KF (Fluorure de potassium) (Acros réf. 20135).

Le mélange a été laissé incuber sur la nuit à 4 °C avant lecture de la fluorescence (λₑₓ 337 nm/λₑₘ 665 nm, et normalisation à λₑₘ 620 nm) au moyen d'un lecteur PHERASTAR (BMG).

Le calcul du résultat est effectué comme suit.

La moyenne des ratios λₑₘ 665/ λₑₘ 620 des contrôles positifs est effectuées : S.

La moyenne des ratios λₑₘ 665/ λₑₘ 620 des contrôles négatifs est effectuées : B.

La moyenne des ratios λₑₘ 665/ λₑₘ 620 pour chaque point de la gamme de concentrations des composés testés est effectuées : C.

Pour chaque point de la gamme de concentrations des composés testés, un pourcentage d'activité A est déterminé par la formule : A = (C - B) / (S - B).

Une courbe dose-réponse est établie avec le logiciel GraphPad Prism® V.5.02 en utilisant l'équation d'une courbe dose-réponse sigmoïdale à coefficient de pente variable, selon les indications du logiciel.

L'IC₅₀ (concentration inhibitrice de 50 % de l'effet) ou l'EC₅₀ (concentration activatrice de 50 % de l'effet) sont déterminées en prenant la concentration à mi hauteur, entre le bas et la phase plateau de la courbe obtenue.

Une diminution de A est indicative de la présence de composés aptes à prévenir ou déstabiliser l'interaction entre la SASPase et la FLG2.

Une augmentation de A est indicative de la présence de composés aptes à stabiliser ou favoriser l'interaction entre la SASPase et la FLG2.

Le criblage d'une banque de molécules HBGXS10 (ChemDiv Discovery Chemistry Collection Public Database (Order Number: 4477-2039), ChemDiv, Inc. San Diego, USA) a permis d'identifier le composé, dit « M7 », de formule (II) suivante :

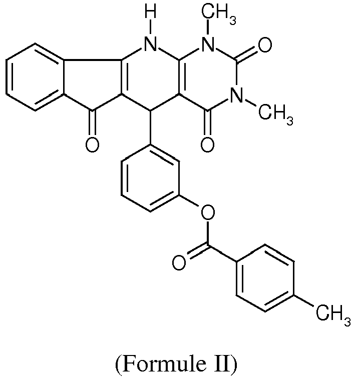

Ce composé a conduit à une inhibition de l'interaction SASPase-FLG2 et présente une IC₅₀ d'environ 2 µM.

Le protocole de criblage de l'invention s'avère donc particulièrement avantageux pour identifier de nouveaux composés actifs aptes à moduler positivement ou négativement l'interaction SASPase-FLG2.

Les nouveaux composés sont susceptibles d'être avantageusement utilisés à l'égard de défauts esthétiques ou de troubles pathologiques de la peau et de ses annexes résultant d'un déséquilibre de la prolifération et/ou de la différenciation des cellules de l'épiderme, et en particulier le vieillissement et la sécheresse cutanée.

### Exemple 3

### Effet du composé « M7 » sur la différentiation et/ou la prolifération des cellules d'un épiderme

### a- Matériels et méthodes

Un modèle d'épiderme reconstruit humain Episkin J6 (référence 09-EPIS-015) a été cultivé par émersion en milieu de différenciation Episkin, 3,5ml sous insert en étuve à 37 °C, 5 % CO₂ et le milieu étant changé tous les 2 jours.

Le composé « M7 » a été testé aux concentrations de 10 µM, 1 µM et 0,5 µM par incubation du modèle d'épiderme avec un milieu de culture de différenciation fourni par EpiSkin®, comprenant le composé à la concentration testé ou une solution tampon à titre de contrôle (DMSO à 0,1 % final).

L'effet du composé « M7 » sur la différentiation et/ou la prolifération des cellules de l'épiderme est évalué par la mesure de l'épaisseur du *stratum corneum*, la mesure de l'expression et/ou de l'activation de la Transglutaminase I (TGI), de la Caspase 14, de la SASPase, de la Cornéodésmosine, de la Desmogléine 1 ou de la protéine Ki67.

L'épaisseur du *stratum corneum* a été estimée par observation histologique, sur coupe.

L'activation, l'expression et la maturation de la TGI, de la Filaggrine, de la Caspase 14, de la SASPase, de la Cornéodésmosine et de la Desmogléine 1 ont été déterminées par Western Blot.

L'activation, l'expression et la maturation de Ki67 ont été déterminées par Immunofluorescence sur coupes congelées.

Pour le Western Blot, les protéines solubles, TGI, Caspase 14, Corneodesmosine et Saspase ont été extraites à partir de deux nacelles Episkin® au potter manuel dans 1 ml de tampon dit « natif + » (TBS, 1M NaCl, 0,1 % Tween 20). L'extrait est centrifugé à 14000 rpm, à 4 °C pendant 10 min. Le surnageant obtenu est filtré sur filtre Millipore 0.45µm (millex) puis les protéines sont quantifiées par la méthode BCA (Pierce). Les concentrations en protéines sont équilibrées à 625µg/ml final.

Les extraits sont déposés sur un gel Criterion 10-20 % (réf : 345-0044 Bio-Rad) selon le protocole du fournisseur puis transférés sur membrane de PVDF par la méthode semi-sec.

La détection immunochimique du Western-Blot est effectuée selon un protocole classique réalisé avec des anticorps primaires spécifiques de la cible et des secondaires couplés HRP. La révélation se fait par les kits ECL ; ECL plus ; ECL advanced (Amersham) selon la sensibilité nécessaire et en suivant le protocole du fournisseur. L'appareil d'imagerie photonique utilisé est le FluorS Multilmager (Bio-Rad)

Pour la TGI, anticorps primaire : Covalab pab 0061 au 1/100^{e} produit chez le lapin ; anticorps secondaire anti lapin couplé HRP au 1/4000^{e} : Bio-rad 170-6515

Pour la Caspase 14, anticorps primaire : Santa Cruz sc-5628 au 1/500^{e} produit chez le lapin ; anticorps secondaire anti lapin couplé HRP au 1/400^{e} : Bio-rad 170-6515

Pour la Corneodesmosine, anticorps primaire : Abnova 1041-M01 au 1/5000^{e} produit chez la souris ; anticorps secondaire anti souris couplé HRP au 1/4000^{e} : Bio-rad 170-6516

Pour la SASPase, anticorps primaire : Covalab 7H9a105 au 1/2000^{e} produit chez la souris ; anticorps secondaire anti souris couplé HRP au 1/4000^{e} : Bio-rad 170-6516

Pour le Western Blot de la Filaggrine et de la Desmogleine 1, ces protéines sont extraites du culot obtenu lors de l'extraction des protéines dites solubles. Les culots sont broyés au potter électrique dans 200 µl de tampon Laemmli complet (Tris 0,0625mM ; SDS 2 % ; DTT 200mM ; glycerol 10 %) puis chauffés à 90 °C 10 min et enfin centrifugés 10 min à 14000 rpm à température ambiante.

Les surnageant obtenus sont dosés en protéines par la méthode Bradford (Cytoskeleton). Les concentrations en protéines totales sont équilibrées à 1 mg/ml final. Un protocole de Western Blot identique au précédent est appliqué.

Pour la Filaggrine, anticorps primaire : Abcam ab24584 au 1/1000^{e} produit chez le lapin ; anticorps secondaire anti lapin couplé HRP au 1/4000^{e} : Bio-rad 170-6515

Pour la Desmogleine 1, anticorps primaire : Progen 61002 au 1/100^{e} produit chez la souris ; anticorps secondaire anti souris couplé HRP au 1/4000^{e} : Bio-rad 170-6516

Le marquage en immunofluorescence sur coupes congelées est réalisé au moyen d'un protocole standard, avec un tampon PBS ; BSA 0,2 % pour les étapes de lavage et d'incubation des anticorps primaires et secondaire, et avec un tampon PBS ; BSA 5 % pour l'étape de blocage des sites non spécifiques.

Pour la détection du Ki67, anticorps primaire : Novocastra mm1 au 1/20^{e} produit chez la souris ; anticorps secondaire anti souris couplé AlexaFLuor A488 au 1/1000^{e} (Molecular Probes A11017). L'appareil de visualisation et de prise d'images est un microscope optique LEICA®.

### b- Résultats

L'application du composé « M7 » a conduit à un épaississement du *stratum corneum,* observable en coupe histologique par microscopie optique, dès la plus faible concentration testée.

Une augmentation de l'expression et de l'activation de la TGI, de la Caspase 14, de la SASPase, de la Cornéodésmosine et de la Désmogléine a été observée dans l'épiderme Episkin cultivé en présence de « M7 ».

L'expression de ces protéines est associée à la différenciation des cellules de l'épiderme.

Une diminution de l'activation et de l'expression de la protéine Ki67 a également été observée. Cette protéine est associée à l'arrêt de la prolifération des cellules épidermiques.

L'épaississement du *stratum corneum* et l'augmentation de l'expression et de l'activation des protéines transglutaminase I, Caspase 14, SASPase, Cornéodésmosine, Désmogléine sont caractéristiques d'une différenciation tardive de l'épiderme.

Le composé « M7 » s'avère donc être doté de la propriété de moduler la prolifération et la différentiation des cellules d'un épiderme.

Un tel composé s'avère donc être particulièrement intéressant pour la prévention ou le traitement de désordres pathologiques ou de défauts esthétiques de la peau et/ou de ses annexes impliquant un déséquilibre de la différenciation et/ou de la prolifération des cellules de l'épiderme.

## Revendications

1. Utilisation cosmétique non-thérapeutique d'au moins un composé apte à moduler l'interaction entre une première et une seconde protéines partenaires, des homologues, ou des fragments desdites protéines, lesdites première et seconde protéines étant la Skin Aspartic Protease, ou SASPase, et la Filaggrine-2, ou FLG2, à titre d'agent actif pour traiter et/ou prévenir un défaut esthétique de la peau et/ou de ses annexes lié à un déséquilibre de la différentiation et/ou de la prolifération des cellules d'un épiderme,
ledit composé étant représenté par les formules générales (Ia) ou (Ib) suivantes : 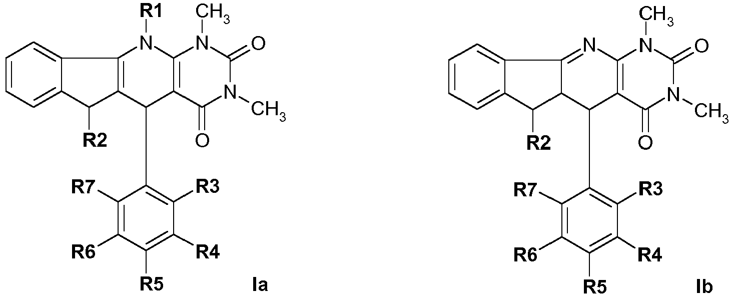 dans lesquelles :
- R¹ représente H, un alkyle en C₁-C₄, linéaire ou ramifié, saturé ou insaturé, ou -C(O)R⁸, dans lequel R⁸ représente un alkyle en C₁-C₄, linéaire ou ramifié, saturé ou insaturé, R¹ représentant de préférence H, un méthyle, un éthyle, un propyle, ou -C(O)R⁸ dans lequel R⁸ représente un méthyle, un éthyle ou un propyle ;
- R² représente =O, -OR⁹ ou -OC(O)R⁹, dans lesquels R⁹ représente H ou un alkyle saturé en C₁-C₂, R² représentant de préférence =O ou -OR⁹, préférentiellement =O ;
- R³, R⁴, R⁵, R⁶, R⁷ représentent, indépendamment l'un de l'autre, H ; -NO₂ ; -OH ; un fluor ; -CF₃ ; un alkyle en C₁-C₄, linéaire ou ramifié, saturé ou insaturé ; un phényle ; -OC(O)-CH(Ph)₂ ; -O-Ph-X avec X représentant H, -OH, -NO₂, un fluor, un alkyle ou un alkoxy en C₁-C₄, linéaire ou ramifié, saturé ou insaturé ; -O-S(O₂)-Ph-Z avec Z représentant un alkyle en C₁-C₄, linéaire ou ramifié, saturé ou insaturé ; -R⁸OH ; -OR⁹ ; -OC(O)R⁹ avec R⁸ et R⁹ étant tels que définis précédemment ; -R¹⁰Ph avec R¹⁰ étant un alkylène en C₁-C₄, linéaire ou ramifié, saturé ou insaturé ; ou -OC(O)-Yₙ-Ar₁-(X)ₘ avec
- n figurant 0 ou 1 et m figurant un entier variant de 0 à 4, sous réserve que n et m ne figurent pas simultanément 0, Y représentant un alkylène en C₁-C₄, linéaire ou ramifié, saturé ou insaturé, X étant tel que défini précédemment, et Ar₁ figurant 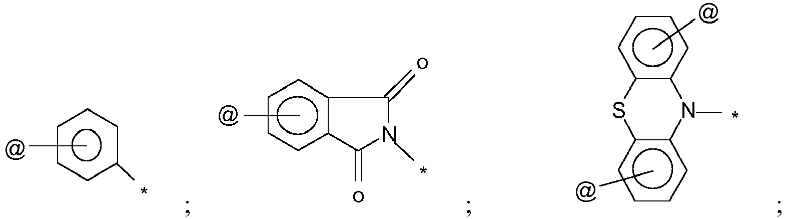 ou 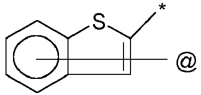 avec * figurant une liaison avec Y et @ figure une liaison avec X,
et leurs sels physiologiquement acceptables,
les homologues ayant une séquence présentant une identité d'au moins 85 % avec l'une des protéines partenaires et une activité biologique de même nature,
les fragments étant des portions d'une protéine partenaire comprenant de 6 à 260 acides aminés contigus et étant dotés d'une activité biologique de même nature.

2. Utilisation selon la revendication 1, dans laquelle le défaut esthétique est choisi parmi des signes cutanés du vieillissement, un désordre de la fonction barrière de l'épiderme, des signes de sécheresse cutanée, un désordre de la desquamation de la peau, un défaut de cicatrisation, et/ou de ré-épithélialisation.

3. Utilisation selon la revendication 1, dans laquelle le désordre est choisi parmi une hypersudation et/ou un problème d'odeur corporelle.

4. Utilisation cosmétique non-thérapeutique d'au moins un composé apte à moduler l'interaction entre une première et une seconde protéines partenaires, des homologues, ou des fragments desdites protéines, lesdites première et seconde protéines étant la Skin Aspartic Protease, ou SASPase, et la Filaggrine-2, ou FLG2, à titre d'agent actif pour favoriser la ré-épithélialisation après un traitement de gommage cutané ou exfoliant, ledit composé étant tel que défini en revendication 1,
les homologues ayant une séquence présentant une identité d'au moins 85 % avec l'une des protéines partenaires et une activité biologique de même nature,
les fragments étant des portions d'une protéine partenaire comprenant de 6 à 260 acides aminés contigus et étant dotés d'une activité biologique de même nature.

5. Utilisation cosmétique non-thérapeutique d'au moins un composé apte à moduler l'interaction entre une première et une seconde protéines partenaires, des homologues, ou des fragments desdites protéines, lesdites première et seconde protéines étant la Skin Aspartic Protease, ou SASPase, et la Filaggrine-2, ou FLG2, à titre d'agent actif pour prévenir et/ou traiter la chute des cheveux et/ou favoriser la repousse des cheveux, ou pour prévenir la repousse des poils, ledit composé étant tel que défini en revendication 1,
les homologues ayant une séquence présentant une identité d'au moins 85 % avec l'une des protéines partenaires et une activité biologique de même nature,
les fragments étant des portions d'une protéine partenaire comprenant de 6 à 260 acides aminés contigus et étant dotés d'une activité biologique de même nature.

6. Utilisation selon l'une quelconque des revendications précédentes, dans laquelle R³, R⁴, R⁵, R⁶ et R⁷ représentent, indépendamment l'un de l'autre, H ; -OH ; un fluor ; un méthyle ou un éthyle ; un phényle ; -OC(O)-CH(Ph)₂ ; -O-Ph-X avec X représentant H,-OH, un fluor, un méthyle, un éthyle, un méthoxy ou un éthoxy ; -O-S(O₂)-Ph-Z avec Z représentant un méthyle ou un éthyle ; -R⁸OH ; -OR⁹ ; -OC(O)R⁹ avec R⁸ représentant un méthyle ou un éthyle et R⁹ représentant H ou un méthyle ; -R¹⁰Ph avec R¹⁰ étant un méthylène ou un éthylène, ou -OC(O)-Yₙ-Ar₁-(X)ₘ avec n, Y, X et Ar₁ étant tels que définis en revendications 1.

7. Utilisation selon l'une quelconque des revendications précédentes, dans laquelle au plus un de R³, R⁴, R⁵, R⁶ et R⁷ représente un -OC(O)-Yₙ-Aₙ-Xₘ, avec Y, n, Ar₁, X et m étant tels que définis en revendication 1, et en particulier R⁴ ou R⁶ représentent -OC(O)-Yₙ-Ar₁-Xₘ avec Y, n, Ar₁, X et m étant tels que définis en revendication 1, de préférence n étant égal à 0, m étant égal à 1, X étant un méthyle ou un méthoxy, préférentiellement un méthyle, et Ar₁ étant 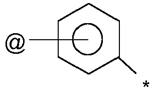 avec * figurant une liaison avec Y et @ figurant une liaison avec X.

8. Utilisation selon l'une quelconque des revendications précédentes, dans laquelle R³, R⁵, R⁷ et R⁴ ou R⁶, représentent, indépendamment l'un de l'autre, H ; -NO₂ ; -OH ; un fluor ; un méthyle ou un éthyle ; un phényle; -R⁸OH ; -OR⁹ ; -OC(O)R⁹ avec R⁸ représentant un méthyle ou un éthyle et R⁹ représentant H ou un méthyle ; -R¹⁰Ph avec R¹⁰ étant un méthylène ou un éthylène.

9. Utilisation selon l'une quelconque des revendications précédentes, dans laquelle ledit composé est représenté par la formule (II) suivante :

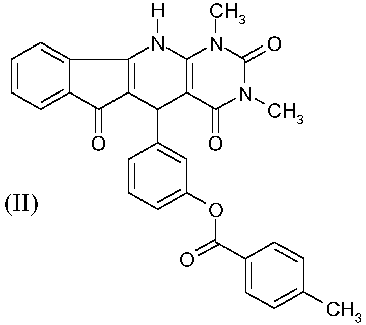

10. Utilisation *in vitro* ou *ex vivo* d'au moins un complexe protéique comprenant une première et une seconde protéines partenaires interagissant l'une avec l'autre, lesdites première et seconde protéines étant la Skin Aspartic Protease, ou SASPase, et la Filaggrine-2, ou FLG2, des homologues, ou des fragments desdites protéines pour cribler des composés aptes à réguler la prolifération et/ou la différenciation des cellules d'un épiderme par modulation de l'interaction entre lesdites première et seconde protéines,
les homologues ayant une séquence présentant une identité d'au moins 85 % avec l'une des protéines partenaires et une activité biologique de même nature,
les fragments étant des portions d'une protéine partenaire comprenant de 6 à 260 acides aminés contigus et étant dotés d'une activité biologique de même nature.

11. Procédé de criblage *in vitro* ou *ex vivo* d'un composé apte à réguler la prolifération et/ou la différenciation des cellules d'un épiderme par modulation de l'interaction entre une première et une seconde protéines partenaires, des homologues, ou des fragments desdites protéines, lesdites première et seconde protéines étant la Skin Aspartic Protease, ou SASPase, et la Filaggrine-2, ou FLG2, ledit procédé comprenant au moins les étapes consistant à :
a) disposer lesdites première et seconde protéines dans des conditions propices à l'interaction entre les première et seconde protéines,
b) associer, préalablement ou postérieurement à l'étape a), au moins une des première et seconde protéines à une entité,
ladite protéine formant avec ladite entité un ensemble apte à émettre un signal après exposition à une longueur d'onde excitatrice, et la réalisation des étapes a) et b) conduisant à l'obtention d'un complexe par interaction desdites première et seconde protéines partenaires,
c) soumettre ledit complexe à ladite longueur d'onde excitatrice pour obtenir un premier signal S1 caractéristique dudit complexe,
d) déterminer quantitativement ou qualitativement ledit signal S1,
e) mettre en contact le complexe avec un milieu présumé contenir un composé à cribler dans des conditions propices à une interaction entre ledit composé et ledit complexe,
f) soumettre le milieu obtenu à l'étape e) à ladite longueur d'onde excitatrice pour obtenir un second signal S2,
g) déterminer quantitativement ou qualitativement ledit signal S2, et
h) comparer les premier et second signaux S1 et S2 pour tirer une conclusion relative à une éventuelle modulation de l'interaction entre les première et seconde protéines par le composé criblé,
les homologues ayant une séquence présentant une identité d'au moins 85 % avec l'une des protéines partenaires et une activité biologique de même nature,
les fragments étant des portions d'une protéine partenaire comprenant de 6 à 260 acides aminés contigus et étant dotés d'une activité biologique de même nature.

12. Procédé selon la revendication 11, dans lequel ladite entité est choisie parmi un support convenant à la résonance plasmonique de surface, un marqueur fluorescent, un anticorps ou une combinaison d'anticorps primaire et secondaire, ledit anticorps ou ledit anticorps secondaire portant un marqueur fluorescent.

13. Procédé selon la revendication 11 ou 12, dans lequel au moins une des première et seconde protéines porte un marqueur d'affinité reconnu par un anticorps, de préférence ledit marqueur étant choisi parmi un marqueur Myc, FLAF, His, His-tag, GST (Glutathion S-Transférase), THX (Thiorédoxine), MBP (Maltose-Binding Protein), THX-His-tag ou THX-His-S-tag.

14. Procédé de criblage selon l'une quelconque des revendications 11 à 13, dans lequel la première et la seconde protéines sont associées, respectivement, à une première et une seconde entités, lesdites entités comportant, respectivement, un groupe fluorescent A et un groupe fluorescent B, lesdits groupes A et B définissant un couple accepteur-donneur d'énergie de fluorescence convenant à la mise en oeuvre d'un transfert d'énergie par résonance de fluorescence.

15. Procédé selon l'une quelconque des revendications 11 à 13, dans lequel ledit composé criblé est apte à traiter et/ou prévenir un désordre esthétique ou un désordre pathologique de la peau et/ou de ses annexes lié à un défaut de la différentiation et/ou de la prolifération des cellules de l'épiderme.

16. Composition pharmaceutique ou dermatologique comprenant dans un milieu physiologiquement acceptable au moins une quantité efficace d'au moins un composé tel que défini selon l'une quelconque des revendications 1 à 9, pour prévenir et/ou traiter un désordre pathologique de la peau et/ou de ses annexes lié à un déséquilibre de différentiation et/ou de prolifération des cellules d'un épiderme.

17. Composition cosmétique comprenant, à titre d'agent actif, dans un milieu physiologiquement acceptable au moins une quantité efficace d'au moins un composé tel que défini selon l'une quelconque des revendications 1 à 9, et au moins un ingrédient cosmétiquement ou dermatologiquement acceptable choisi parmi des actifs cosmétiques ou dermatologiques, des conservateurs, des gélifiants hydrophiles ou lipophiles, des agents tensioactifs non ioniques, anioniques, cationiques, des antioxydants, des sels, des parfums, des charges, des absorbeurs d'odeur, des matières colorantes, des polymères filmogènes, des polymères semi-cristallins, des gommes, des huiles volatiles ou non volatiles, et leurs mélanges.

18. Article de conditionnement d'une composition cosmétique, pharmaceutique ou dermatologique, contenant au moins une composition cosmétique, pharmaceutique ou dermatologique comprenant au moins un composé tel que défini selon l'une quelconque des revendications 1 à 9.

19. Procédé cosmétique pour prévenir et/ou traiter un défaut esthétique de la peau et/ou de ses annexes lié à un déséquilibre de la différentiation et/ou de la prolifération des cellules de l'épiderme chez un individu, comprenant au moins une étape d'administration audit individu d'au moins un composé tel que défini selon l'une quelconque des revendications 1 à 9.

20. Complexe protéique comprenant une première et une seconde protéines partenaires interagissant l'une avec l'autre, lesdites première et seconde protéines étant la Skin Aspartic Protease, ou SASPase, et la Filaggrine-2, ou FLG2, des homologues, ou des fragments desdites protéines
les homologues ayant une séquence présentant une identité d'au moins 85 % avec l'une des protéines partenaires et une activité biologique de même nature,
les fragments étant des portions d'une protéine partenaire comprenant de 6 à 260 acides aminés contigus et étant dotés d'une activité biologique de même nature.

## Patentansprüche

1. Kosmetische nicht therapeutische Verwendung von mindestens einer Verbindung, die dazu geeignet ist, die Wechselwirkung zwischen einem ersten und einem zweiten Partnerprotein, Homologen oder Fragmenten der Proteine zu modulieren, wobei die ersten und zweiten Proteine Skin Aspartic Protease oder SASPase und Filaggrin-2 oder FLG2 sind, als Wirkstoff zur Behandlung und/oder Prävention eines Schönheitsfehlers der Haut und/oder ihrer Anhänge in Verbindung mit einem Ungleichgewicht der Differenzierung und/oder der Proliferation der Zellen einer Epidermis,
wobei die Verbindung durch die folgenden allgemeinen Formeln (la) oder (Ib) dargestellt ist: 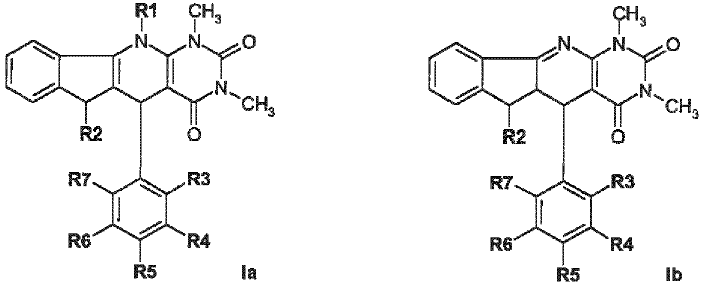 wobei
- R¹ H, ein lineares oder verzweigtes, gesättigtes oder ungesättigtes C₁-C₄-Alkyl, oder - C(O)R⁸ darstellt, wobei R⁸ ein lineares oder verzweigtes, gesättigtes oder ungesättigtes C₁-C₄-Alkyl darstellt, wobei R¹ vorzugsweise H, ein Methyl, ein Ethyl, ein Propyl, oder -C(O)R⁸ darstellt, wobei R⁸ ein Methyl, ein Ethyl oder ein Propyl darstellt;
- R² =O, -OR⁹ oder -OC(O)R⁹ darstellt, wobei R⁹ H oder ein gesättigtes C₁-C₂-Alkyl darstellt, R² vorzugsweise =O oder -OR⁹, bevorzugt =O darstellt;
- R³, R⁴, R⁵, R⁶, R⁷ unabhängig voneinander H; -NO₂; -OH; ein Fluor; -CF₃; ein lineares oder verzweigtes, gesättigtes oder ungesättigtes C₁-C₄-Alkyl; ein Phenyl; -OC(O)-CH(Ph)₂; -O-Ph-X, wobei X H, -OH, -NO₂, ein Fluor, ein Alkyl oder ein lineares oder verzweigtes, gesättigtes oder ungesättigtes C₁-C₄-Alkoxy darstellt; -O-S(O₂)-Ph-Z, wobei Z ein lineares oder verzweigtes, gesättigtes oder ungesättigtes C₁-C₄-Alkyl darstellt,; -R⁸OH; -OR⁹; -OC(O)R⁹, wobei R⁸ und R⁹ wie zuvor definiert sind; -R¹⁰Ph, wobei R¹⁰ für ein lineares oder verzweigtes, gesättigtes oder ungesättigtes C₁-C₄-Alkylen steht; oder -OC(O)-Yₙ-Ar₁-(X)ₘ darstellt, darstellen, wobei
- n 0 oder 1 darstellt und m eine ganze Zahl von 0 bis 4 darstellt, mit der Maßgabe, dass n und m nicht gleichzeitig 0 sind, Y ein lineares oder verzweigtes, gesättigtes oder ungesättigtes C₁-C₄-Alkylen darstellt, X wie zuvor definiert ist, und Ar₁ folgendes darstellt 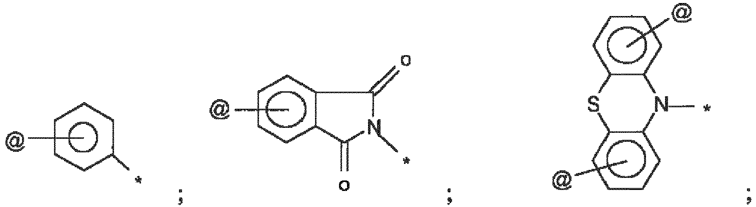 oder 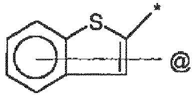
wobei * eine Bindung mit Y darstellt und @ eine Bindung mit X darstellt, und ihre physiologisch verträglichen Salze,
wobei die Homologe eine Sequenz aufweisen, die eine Identität von mindestens 85 % mit einem der Partnerproteine und eine biologische Aktivität der gleichen Art zeigt,
die Fragmente Teile eines Partnerproteins sind, umfassend von 6 bis 260 zusammenhängende und mit einer biologischen Aktivität der gleichen Art ausgestattete Aminosäuren.

2. Verwendung nach Anspruch 1, wobei der Schönheitsfehler ausgewählt ist aus Anzeichen für Hautalterung, einer Störung der Barrierefunktion der Epidermis, Anzeichen für Hauttrockenheit, einer Störung der Abschuppung der Haut, einem Fehler der Narbenbildung, und/oder der Reepithelisierung.

3. Verwendung nach Anspruch 1, wobei die Störung ausgewählt ist aus einer Hypersudation und/oder einem Körpergeruchsproblem.

4. Kosmetische nicht therapeutische Verwendung von mindestens einer Verbindung, die dazu geeignet ist, die Wechselwirkung zwischen einem ersten und einem zweiten Partnerprotein, Homologen oder Fragmenten der Proteine zu modulieren, wobei die ersten und einem zweiten Proteine Skin Aspartic Protease oder SASPase und Filaggrine-2 oder FLG2 sind, als Wirkstoff zur Begünstigung der Reepithelisierung nach einer Abrasions- oder Peelingbehandlung, wobei die Verbindung wie in Anspruch 1 definiert ist,
wobei die Homologe eine Sequenz aufweisen, die eine Identität von mindestens 85 % mit einem der Partnerproteine und eine biologische Aktivität der gleichen Art zeigt,
die Fragmente Teile eines Partnerproteins sind, umfassend von 6 bis 260 zusammenhängende und mit einer biologischen Aktivität der gleichen Art ausgestattete Aminosäuren.

5. Kosmetische nicht therapeutische Verwendung von mindestens einer Verbindung, die dazu geeignet ist, die Wechselwirkung zwischen einem ersten und einem zweiten Partnerprotein, Homologen oder Fragmenten der Proteine zu modulieren, wobei die ersten und zweiten Proteine Skin Aspartic Protease oder SASPase und Filaggrin-2 oder FLG2 sind, als Wirkstoff zur Prävention und/oder Behandlung von Haarausfall und/oder zur Förderung des Haarwachstums, oder zur Prävention des Nachwachsens von Körperhaaren, wobei die Verbindung wie in Anspruch 1 definiert ist,
wobei die Homologe eine Sequenz aufweisen, die eine Identität von mindestens 85 % mit einem der Partnerproteine und eine biologische Aktivität der gleichen Art zeigt,
die Fragmente Teile eines Partnerproteins sind, umfassend von 6 bis 260 zusammenhängende und mit einer biologischen Aktivität der gleichen Art ausgestattete Aminosäuren.

6. Verwendung nach einem der vorangehenden Ansprüche , wobei R³, R⁴, R⁵, R⁶ und R⁷ unabhängig voneinander H; -OH; ein Fluor; ein Methyl oder ein Ethyl; ein Phenyl; -OC(O)-CH(Ph)₂; -O-Ph-X, wobei X H, -OH, ein Fluor, ein Methyl, ein Ethyl, ein Methoxy oder ein Ethoxy darstellt; -O-S(O₂)-Ph-Z, wobei Z ein Methyl oder ein Ethyl darstellt; -R⁸OH; -OR⁹;-OC(O)R⁹, wobei R⁸ ein Methyl oder ein Ethyl darstellt und R⁹ H oder ein Methyl darstellt,;-R¹⁰Ph, wobei R¹⁰ für ein Methylen oder ein Ethylen steht, oder -OC(O)-Yₙ-Ar₁-(X)ₘ, wobei n, Y, X und Ar₁ wie in Anspruch 1 definiert sind, darstellen.

7. Verwendung nach einem der vorangehenden Ansprüche , wobei höchstens eines von R³, R⁴, R⁵, R⁶ und R⁷ ein -OC(O)-Yn-Ar₁-Xm darstellt, wobei Y, n, Ar₁, X und m wie in Anspruch 1 definiert sind, und insbesondere R⁴ oder R⁶-OC(O)-Yn-Ar₁-Xₘ darstellt, wobei Y, n, Ar₁, X und m wie in Anspruch 1 definiert sind, wobei n vorzugsweise gleich 0 ist, m gleich 1 ist, X für ein Methyl oder ein Methoxy, bevorzugt ein Methyl steht, und Ar₁ 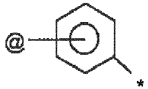 darstellt, wobei * für eine Bindung steht, wobei Y und @ für eine Bindung mit X stehen.

8. Verwendung nach einem der vorangehenden Ansprüche , wobei R³, R⁵, R⁷ und R⁴ oder R⁶ unabhängig voneinander H; -NO₂; -OH; ein Fluor; ein Methyl oder ein Ethyl; ein Phenyl; - R⁸OH; -OR⁹; -OC(O)R⁹, wobei R⁸ ein Methyl oder ein Ethyl darstellt, und R⁹ H oder ein Methyl darstellt; -R¹⁰Ph, wobei für ein R¹⁰ ein Methylen oder ein Ethylen steht, darstellen.

9. Verwendung nach einem der vorangehenden Ansprüche , wobei die Verbindung durch die folgende Formel (II) dargestellt ist:

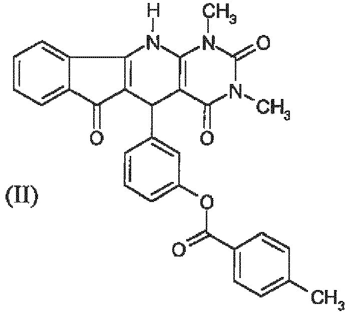

10. Verwendung, in vitro oder ex vivo, von mindestens einem Proteinkomplex, umfassend ein erstes und ein zweites Partnerprotein, die miteinander in Wechselwirkung stehen, wobei das erste und das zweite Protein Skin Aspartic Protease, oder SASPase und Filaggrin-2 oder FLG2, Homologe, oder Fragmente der Proteine sind, zum Screening von Verbindungen, die zur Regulierung der Proliferation und/oder der Differenzierung der Zellen einer Epidermis durch Modulation der Wechselwirkung zwischen dem ersten und dem zweiten Protein geeignet sind,
wobei die Homologe eine Sequenz aufweisen, die eine Identität von mindestens 85 % mit einem der Partnerproteine und eine biologische Aktivität der gleichen Art zeigt,
die Fragmente Teile eines Partnerproteins sind, umfassend von 6 bis 260 zusammenhängende und mit einer biologischen Aktivität der gleichen Art ausgestattete Aminosäuren.

11. Verfahren zum Screening, in vitro oder ex vivo, einer Verbindung die dazu geeignet ist, die Proliferation und/oder die Differenzierung der Zellen einer Epidermis durch Modulation der Wechselwirkung zwischen einem ersten und einem zweiten Partnerprotein, Homologen oder Fragmenten der Proteine zu regulieren, wobei das erste und das zweite Protein Skin Aspartic Protease oder SASPase und Filaggrin-2 oder FLG2 sind, wobei das Verfahren mindestens die Schritte umfasst, die bestehen aus:
a) Vorlegen des ersten und des zweiten Proteins unter Bedingungen, die für die Wechselwirkung zwischen dem ersten und einem zweiten Protein günstig sind,
b) Kombinieren, vor oder nach Schritt a), von mindestens einem des ersten und des zweiten Proteins zu einer Einheit,
wobei das Protein mit der Einheit ein Ganzes bildet, das dazu geeignet ist, nach der Exposition gegenüber einer Anregungswellenlänge ein Signal auszusenden, und wobei die Umsetzung der Schritte a) und b) zum Erhalt eines Komplexes durch Wechselwirkung des ersten und des zweiten Partnerproteins führen,
c) Unterziehen des Komplexes der Anregungswellenlänge, um ein erstes für den Komplex charakteristisches Signal S1 zu erhalten,
d) Bestimmen, quantitativ oder qualitativ, des Signals S1,
e) Inkontaktbringen des Komplexes mit einem Medium, das vermutlich eine zu screenende Verbindung enthält, unter Bedingungen, die für eine Wechselwirkung zwischen der Verbindung und dem Komplex geeignet sind,
f) Unterziehen des in Schritt e) erhaltenen Mediums der Anregungswellenlänge, um ein zweites Signal S2 zu erhalten,
g) Bestimmen, quantitativ oder qualitativ, des Signals S2, und
h) Vergleichen der ersten und zweiten Signale S1 und S2, um eine Schlussfolgerung im Hinblick auf eine mögliche Modulation der Wechselwirkung zwischen dem ersten und dem zweiten Protein durch die gescreente Verbindung zu ziehen,
wobei die Homologe eine Sequenz aufweisen, die eine Identität von mindestens 85 % mit einem der Partnerproteine und eine biologische Aktivität der gleichen Art zeigt,
die Fragmente Teile eines Partnerproteins sind, umfassend von 6 bis 260 zusammenhängende und mit einer biologischen Aktivität der gleichen Art ausgestattete Aminosäuren.

12. Verfahren nach Anspruch 11, wobei die Einheit ausgewählt ist aus einem Träger, der zur Oberflächen-Plasmonresonanz geeignet ist, einem Fluoreszenzmarker, einem Antikörper oder einer Kombination von primären und sekundären Antikörpern, wobei der Antikörper oder der sekundäre Antikörper einen Fluoreszenzmarker trägt.

13. Verfahren nach Anspruch 11 oder 12, wobei mindestens eines des ersten und zweiten Proteins einen Affinitätsmarker trägt, der von einem Antikörper erkannt wird, wobei der Marker vorzugsweise ausgewählt ist aus einem Marker Myc, FLAF, His, His-tag, GST (Glutathion-S-Transferase), THX (Thioredoxin), MBP (Maltose-Bindungsprotein), THX-His-tag oder THX-His-S-tag.

14. Verfahren zum Screening nach einem der Ansprüche 11 bis 13, wobei das erste und das zweite Protein jeweils mit einer ersten und einer zweiten Einheit kombiniert werden, wobei die Einheiten jeweils eine fluoreszierende Gruppe A und eine fluoreszierende Gruppe B umfassen, wobei die Gruppen A und B ein Fluoreszenzenergie-Akzeptor-Donor-Paar definieren, das zur Durchführung einer Energieübertragung durch Fluoreszenzresonanz geeignet ist.

15. Verfahren nach einem der Ansprüche 11 bis 13, wobei die gescreente Verbindung zur Behandlung und/oder Prävention einer ästhetischen Störung oder einer pathologischen Störung der Haut und/oder ihrer Anhänge geeignet ist, die mit einem Fehler in der Differenzierung und/oder der Proliferation der Epidermiszellen zusammenhängt.

16. Pharmazeutische oder dermatologische Zusammensetzung, umfassend, in einem physiologisch verträglichen Medium, mindestens eine wirksame Menge von mindestens einer Verbindung, wie nach einem der Ansprüche 1 bis 9 definiert, zur Prävention und/oder Behandlung einer pathologischen Störung der Haut und/oder ihrer Anhänge in Verbindung mit einem Ungleichgewicht der Differenzierung und/oder der Proliferation der Zellen einer Epidermis.

17. Kosmetische Zusammensetzung, umfassend als Wirkstoff in einem physiologisch verträglichen Medium, mindestens eine wirksame Menge von mindestens einer Verbindung wie nach einem der Ansprüche 1 bis 9 definiert, und mindestens einen kosmetisch oder dermatologisch verträglichen Bestandteil, der ausgewählt ist aus kosmetischen oder dermatologischen Wirkstoffen, Konservierungsstoffen, hydrophilen oder lipophilen Gelbildnern, nichtionischen, anionischen, kationischen Tensiden, Antioxidantien, Salzen, Duftstoffen, Füllstoffen, Geruchsabsorbern, Farbstoffen, filmbildenen Polymeren, semikristallinen Polymeren, Gummen, flüchtigen oder nicht flüchtigen Ölen, und ihren Gemischen.

18. Artikel zur Konditionierung einer kosmetischen, pharmazeutischen oder dermatologischen Zusammensetzung, enthaltend mindestens eine kosmetische, pharmazeutische oder dermatologische Zusammensetzung, umfassend mindestens eine Verbindung wie nach einem der Ansprüche 1 bis 9 definiert.

19. Kosmetisches Verfahren zur Prävention und/oder Behandlung eines Schönheitsfehlers der Haut und/oder ihrer Anhänge in Verbindung mit einem Ungleichgewicht der Differenzierung und/oder der Proliferation der Zellen der Epidermis in einem Individuum, umfassend mindestens einen Schritt der Verabreichung an das Individuum von mindestens einer Verbindung wie nach einem der Ansprüche 1 bis 9 definiert.

20. Proteinkomplex, umfassend ein erstes und ein zweites Partnerprotein, die miteinander wechselwirken, wobei das erste und das zweite Protein Skin Aspartic Protease oder SASPase und Filaggrin-2 oder FLG2, Homologe oder Fragmente der Proteine sind,
wobei die Homologe eine Sequenz aufweisen, die eine Identität von mindestens 85 % mit einem der Partnerproteine und eine biologische Aktivität der gleichen Art zeigt, wobei die Fragmente Teile eines Partnerproteins sind, umfassend von 6 bis 260 zusammenhängende und mit einer biologischen Aktivität der gleichen Art ausgestattete Aminosäuren.

## Claims

1. Non-therapeutic cosmetic use of at least one compound capable of modulating the interaction between a first and a second partner proteins, homologs or fragments of said proteins, said first and second proteins being Skin Aspartic Protease or SASPase, and Filaggrin-2 or FLG2 as active agent for treating and/or preventing an esthetic defect of the skin and/or its integuments related to an imbalance in epidermal cell differentiation and/or proliferation,
said compound being represented by the following general formulas (Ia) or (Ib): 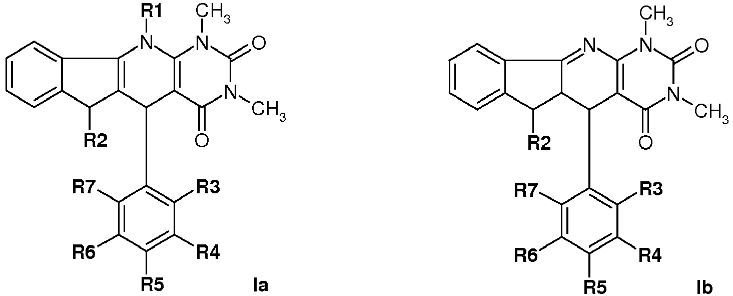 wherein:
- R¹ is H, a linear or branched, saturated or unsaturated C₁-C₄ alkyl, or -C(O)R⁸ where R⁸ is a linear or branched, saturated or unsaturated C₁-C₄ alkyl, R¹ preferably being H, a methyl, ethyl, propyl, or -C(O)R⁸ where R⁸ is a methyl, ethyl or propyl;
- R² is =O, -OR⁹ or -OC(O)R⁹, where R⁹ is H or a saturated C₁-C₂ alkyl, R² preferably being =O or -OR⁹, preferably =0;
- R³, R⁴, R⁵, R⁶, R⁷ are each independently H; -NO₂; -OH; a fluorine; -CF₃; a linear or branched, saturated or unsaturated C₁-C₄ alkyl; a phenyl; -OC(O)-CH(Ph)₂; -O-Ph-X with X representing H, -OH, -NO₂, a fluorine, a linear or branched, saturated or unsaturated C₁-C₄ alkyl or alkoxy; -O-S(O₂)-Ph-Z with Z representing a linear or branched, saturated or unsaturated C₁-C₄ alkyl; -R⁸OH; -OR⁹; -OC(O)R⁹ with R⁸ and R⁹ being as defined previously; -R¹⁰Ph with R¹⁰ being a linear or branched, saturated or unsaturated C₁-C₄ alkylene; or -OC(O)-Yₙ-Ar₁-(X)ₘ with
- n being 0 or 1 and m being an integer ranging from 0 to 4, providing that n and m are not simultaneously 0, Y representing a linear or branched, saturated or unsaturated C₁-C₄ alkylene, X being as defined previously, and Ar₁ being 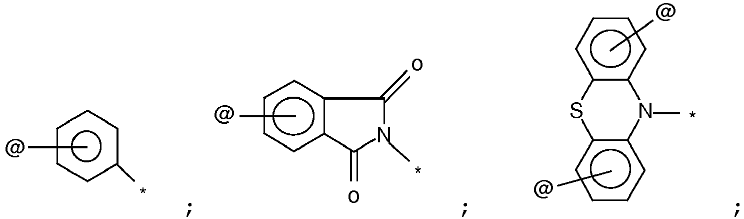 or 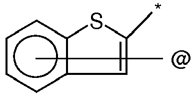 with * being a bond with Y and @ is a bond with X and the physiologically acceptable salts thereof, the homologs displaying a sequence having at least 85 % identity with one of the partner proteins and having biological activity of same nature,
the fragments being portions of a partner protein comprising from 6 to 260 contiguous amino acids and having biological activity of same nature.

2. The use as claimed in claim 1, wherein the esthetic defect is chosen from signs of aging of the skin, a disorder of the epidermal barrier function, signs of skin dryness, a skin desquamation disorder, a cicatrization and/or re-epithelialization defect.

3. The use as claimed in claim 1, in which the disorder is chosen from hypersudation and/or a body odor problem.

4. Non-therapeutic cosmetic use of at least one compound that is capable of modulating the interaction between a first and a second partner proteins, homologs or fragments of said proteins, said first and second proteins being Skin Aspartic Protease or SASPase, and Filaggrin-2 or FLG2, as active agent for promoting re-epithelialization after a cutaneous scrubbing or exfoliant treatment, said compound being such as defined in claim 1,
the homologs displaying a sequence having at least 85 % identity with one of the partner proteins and having biological activity of same nature,
the fragments being portions of a partner protein comprising from 6 to 260 contiguous amino acids and having biological activity of same nature.

5. Non-therapeutic cosmetic use of at least one compound that is capable of modulating the interaction between a first and a second partner proteins, homologs or fragments of said proteins, said first and second proteins being Skin Aspartic Protease or SASPase, and Filaggrin-2 or FLG2, as active agent for preventing and/or treating hair loss and/or promoting hair regrowth, or for preventing body hair regrowth, said compound being such as defined in claim 1,
the homologs displaying a sequence having at least 85 % identity with one of the partner proteins and having biological activity of same nature,
the fragments being portions of a partner protein comprising from 6 to 260 contiguous amino acids and having biological activity of same nature.

6. The use as claimed in any one of the preceding claims, wherein R³, R⁴, R⁵, R⁶ and R⁷ are each independently H; -OH; a fluorine; a methyl or ethyl; a phenyl; -OC(O)-CH(Ph)₂; -O-Ph-X with X representing H, -OH, a fluorine, a methyl, ethyl, methoxy or ethoxy; -O-S(O₂)-Ph-Z with Z representing a methyl or ethyl; -R⁸OH; -OR⁹; -OC(O)R⁹ with R⁸ representing a methyl or ethyl and R⁹ representing H or a methyl; -R¹⁰Ph with R¹⁰ being a methylene or ethylene, or -OC(O)-Yₙ-Ar₁-(X)ₘ with n, Y, X and Ar₁ being such as defined in claim 1.

7. The use as claimed in any of the preceding claims, wherein at most one from among R³, R⁴, R⁵, R⁶ and R⁷ is -OC(O)-Yₙ-Ar₁-Xₘ, with Y, n, Ar₁, X and m being as defined in claim 1, and in particular R⁴ or R⁶ is -OC(O)-Yₙ-Ar₁-Xₘ with Y, n, Ar₁, X and m being as defined in claim 1, preferably n equalling 0, m equalling 1, X being a methyl or methoxy, preferably a methyl, and Ar₁ is: 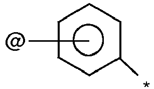 with * being a bond with Y and @ is a bond with X.

8. The use as claimed in any one of the preceding claims, wherein R³, R⁵, R⁷ and R⁴ or R⁶ are each independently H; -NO₂; -OH; a fluorine; a methyl or ethyl; a phenyl; -R⁸OH; -OR⁹; -OC(O)R⁹ with R⁸ representing a methyl or ethyl and R⁹ representing H or a methyl; -R¹⁰Ph with R¹⁰ being a methylene or ethylene.

9. The use as claimed in any of the preceding claims, wherein said compound is represented by following formula (II):

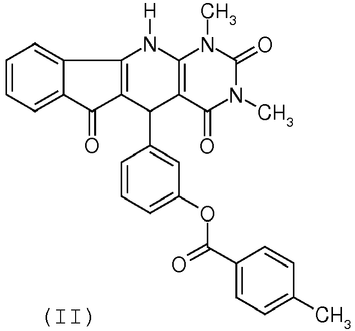

10. *In vitro* or ex vivo use of at least one protein complex comprising a first and a second partner proteins interacting with each other, said first and second proteins being Skin Aspartic Protease or SASPase, and Filaggrin-2 or FLG2, homologs or fragments of said proteins, for screening compounds that are capable of regulating the proliferation and/or differentiation of epidermal cells by modulating the interaction between said first and second proteins,
the homologs displaying a sequence having at least 85 % identity with one of the partner proteins and having biological activity of same nature,
the fragments being portions of a partner protein comprising from 6 to 260 contiguous amino acids and having biological activity of same nature.

11. A process for *in vitro* or *ex vivo* screening of a compound that is capable of regulating the proliferation and/or differentiation of epidermal cells by modulating the interaction between a first and a second partner proteins, homologs or fragments of said proteins, said first and second proteins being Skin Aspartic Protease or SASPase, and Filaggrin-2 or FLG2, said process comprising at least the steps consisting in:
a) placing said first and second proteins under conditions suitable for interaction between the first and second proteins,
b) associating, before or after step a), at least one of the first and second proteins with a species,
said protein forming with said species an assembly capable of emitting a signal after exposure to an excitatory wavelength, and the implementation of steps a) and b) leading to the production of a complex by interaction of said first and second partner proteins,
c) subjecting said complex to said excitatory wavelength in order to obtain a first signal S1 characteristic of said complex,
d) determining quantitatively or qualitatively said signal S1,
e) placing the complex in contact with a medium assumed to contain a compound to be screened under conditions suitable for interaction between said compound and said complex,
f) subjecting the medium obtained at step e) to said excitatory wavelength in order to obtain a second signal S2,
g) determining quantitatively or qualitatively said signal S2, and
h) comparing the first and second signals S1 and S2 in order to draw a conclusion relating to possible modulation of the interaction between the first and second proteins by the screened compound,
the homologs displaying a sequence having at least 85 % identity with one of the partner proteins and having biological activity of same nature,
the fragments being portions of a partner protein comprising from 6 to 260 contiguous amino acids and having biological activity of same nature.

12. The process as claimed in claim 11, wherein said species is chosen from among a substrate suitable for surface plasmon resonance, a fluorescent marker, an antibody or a combination of primary and secondary antibodies, said antibody or said secondary antibody carrying a fluorescent marker.

13. The process as claimed in claim 11 or 12, wherein at least one of the first and second proteins carries an affinity marker recognized by an antibody, preferably said marker being selected from among Myc, FLAF, His, His-tag, GST (glutathione S-transferase), THX (thioredoxin), MBP (maltose-binding protein), THX-His-tag or THX-His-S-tag markers.

14. The screening process as claimed in any one of claims 11 to 13, wherein the first and the second proteins are associated, respectively, with a first and a second species, said species comprising, respectively, a fluorescent group A and a fluorescent group B, said groups A and B defining an acceptor-donor pair having fluorescence energy suitable for performing energy transfer via fluorescence resonance.

15. The screening process as claimed in any of claims 11 to 13, wherein said screened compound is capable of treating and/or preventing an esthetic disorder or a pathological disorder of the skin and/or its integuments associated with an epidermal cell differentiation and/or proliferation defect.

16. A pharmaceutical or dermatological composition comprising, in a physiologically acceptable medium, at least an effective amount of at least one compound as defined according to any one of claims 1 to 9, for preventing and/or treating a pathological disorder of the skin and/or its integuments associated with an imbalance in epidermal cell differentiation and/or proliferation.

17. A cosmetic composition comprising, as active agent in a physiologically acceptable medium, at least an effective amount of at least one compound as defined according to any one of claims 1 to 9, and at least one cosmetically or dermatologically acceptable ingredient chosen from cosmetic or dermatological active agents, preserving agents, hydrophilic or lipophilic gellants, nonionic, anionic or cationic surfactants, antioxidants, salts, fragrances, fillers, odor absorbers, dyestuffs, film-forming polymers, semi-crystalline polymers, gums, volatile or non-volatile oils, and mixtures thereof.

18. An article for packaging a cosmetic, pharmaceutical or dermatological composition, containing at least one cosmetic, pharmaceutical or dermatological composition comprising at least one compound as defined according to any one of claims 1 to 9.

19. A cosmetic process for preventing and/or treating an esthetic defect of the skin and/or its integuments associated with an imbalance in epidermal cell differentiation and/or proliferation in an individual, comprising at least one step of administering to said individual at least one compound as defined according to any one of claims 1 to 9.

20. A protein complex comprising a first and a second partner proteins that interact with each other, said first and second proteins being Skin Aspartic Protease or SASPase, and Filaggrin-2 or FLG2, homologs or fragments of said proteins,
the homologs displaying a sequence having at least 85 % identity with one of the partner proteins and having biological activity of same nature,
the fragment being portions of a partner protein comprising from 6 to 260 contiguous amino acids and having biological activity of same nature.
